# EUROPEAN PATENT SPECIFICATION

(11) **EP 4 508 118 B1**
(45) Date of publication and mention of the grant of the patent: **26.11.2025**
(21) Application number: 23720246.0
(22) Date of filing: 13.04.2023
(51) Int. Cl.: C08G 83/00, A61K 49/18, C07F 9/38, A61K 9/00, A61K 49/00, C07C 235/48, C07F 9/40, A61K 47/00

(54) **DENDRITIC MOLECULES, PROCESS FOR THEIR PREPARATION AND USES THEREOF**
DENDRITISCHE MOLEKÜLE, VERFAHREN ZU IHRER HERSTELLUNG UND IHRE VERWENDUNG
MOLECULES DENDRITIQUES, LEUR PROCEDE DE PREPARATION ET LEURS UTILISATIONS

(30) Priority: 14.04.2022 EP 22305547
(43) Date of publication of application: 19.02.2025
(73) Proprietor: Superbranche, 68420 Hattstatt (FR)
(72) Inventor: FELDER-FLESCH, Delphine, 68420 HATTSTATT (FR); AYELA, Benjamin, 67400 ILLKIRCH GRAFFENSTADEN (FR)
(74) Representative: Dennemeyer & Associates S.A.
(86) International application number: PCT/EP2023/059630
(87) International publication number: WO 2023/198813

(56) References cited:
- EP-A1- 3 125 949
- WALTER AURÉLIE ET AL: "Effect of the Functionalization Process on the Colloidal, Magnetic Resonance Imaging, and Bioelimination Properties of Mono- or Bisphosphonate-Anchored Dendronized Iron Oxide Nanoparticles", vol. 82, no. 4, 1 April 2017 (2017-04-01), pages 647 - 659, XP055930165, ISSN: 2192-6506, Retrieved from the Internet <URL:https://api.wiley.com/onlinelibrary/tdm/v1/articles/10.1002%2Fcplu.201700049> DOI: 10.1002/cplu.201700049
- SHI DA ET AL: "Interfacial Behavior of Oligo(Ethylene Glycol) Dendrons Spread Alone and in Combination with a Phospholipid as Langmuir Monolayers at the Air/Water Interface", MOLECULES, vol. 24, no. 22, 14 November 2019 (2019-11-14), pages 4114, XP055930166, DOI: 10.3390/molecules24224114
- ORG WWW RSC ET AL: "Materials Chemistry B Materials for biology and medicine Validation of a dendron concept to tune colloidal stability, MRI relaxivity and bioelimination of functional nanoparticles", 9 January 2015 (2015-01-09), pages 1459 - 1732, XP055930163, Retrieved from the Internet <URL:https://pubs.rsc.org/en/content/articlepdf/2015/tb/c4tb01954g> [retrieved on 20220611]
- SHI DA ET AL: "Microbubbles decorated with dendronized magnetic nanoparticles for biomedical imaging: effective stabilization via fluorous interactions", vol. 10, no. 10, 31 October 2019 (2019-10-31), pages 2103 - 2115, XP055930164, Retrieved from the Internet <URL:https://www.beilstein-journals.org/bjnano/content/pdf/2190-4286-10-205.pdf> DOI: 10.3762/bjnano.10.205

## Description

### TECHNICAL FIELD

The invention belongs to the field of dendritic molecules.

More particularly, the invention relates to polyfunctional organic dendritic molecules, to a process for preparing the same and to the use thereof, in particular as drug carriers or contrast agents.

### BACKGROUND

Dendrimers and their elementary unit called "dendron", are synthetically produced as monodisperse polymeric nanostructures with a tree-like, highly branched architecture. They are routinely synthesized as tunable "nanostructures" that may be designed and regulated as a function of their size, shape, surface chemistry and interior void space. They are typically 2 to 20 nm in diameter. A variety of structures are available, and each has properties such as polyvalency, self-assembling, electrostatic interactions, chemical stability, low cytotoxicity, and solubility.

Dendrimers and dendrons offer a plethora of applications deriving from the intrinsic properties of polymers but also and especially from their characteristics: on-surface easily accessible functions, porosity, flexibility of the internal branches, presence of functionalized cavities, accessibility to the core, and of course multivalency and cooperativity. They are extremely adaptable materials, with respect to their structure, flexibility, porosity or morphology, which can all be tuned at will. Their applications rely on chemistry (synthesis, analysis, catalysis...), materials sciences (films, layers and hybrids), pharmacology (drugs, medicine), nanosciences (nanoparticles), biology, and medicine (immunology). Dendrimers and dendrons are widely investigated and utilized in biomedical applications, as they have multiple surface functional groups that can be used to target or label for imaging and drug delivery applications.

Dendrimers and dendrons have found application in transdermal drug delivery systems and show potential in gene delivery and for enhancing the oral bioavailability of problematic drugs. The presence of numerous surface groups makes dendrimers suitable carriers for delivering high drug payloads. The interior space of the branched structures can be used to conjugate or encapsulate drugs. They are utilized for delivery vehicles of nonsteroidal anti-inflammatory drugs (NSAIDs), anticancer drugs, and other drugs such as simvastatin, famotidine, or quinolones. Drug-dendrimer conjugates show high solubility, reduced systemic toxicity, and selective accumulation in solid tumors.

The multivalent character of dendrimers and dendrons also positioned these well-defined and hyper connected macromolecules to the foreground in the development of new contrast agents for medical imaging or diagnosis platforms with adjustable retention times and bio distribution properties according to their generation/size, to their flexibility and/or their hydrophilicity. In addition to chemistry, the characterization and the physicochemical properties of these structures were studied in detail.

A dendritic approach as a coating strategy for the design of functional nano-objects is particularly interesting in the field of cancer diagnostics. The appeal of such strategy is due to the unique properties of the dendritic structures which can be chemically tuned to reach ideal biodistribution or highly and efficient targeting efficacies. To improve tumor targeting efficacy and to obtain better *in vivo* imaging properties, several studies explored the multivalency effect of dendrimers or of a dendritic surface functionalization of nanomaterials. Due to their conical-like architecture and focal points, dendritic structures are of particular interest as coatings of ultrasmall nanoparticles (NPs) with very high surface curvature. Certainly, such cone shapes are expected to improve steric resistance to macromolecules such as proteins while preventing better particle agglomeration by comparison with their linear counterparts.

Examples of dendritic molecules bearing either a mono- or a bisphosphonate group are provided in the articles by Walter A., et al., entitled "Effect of the Functionalization Process on the Colloidal, Magnetic Resonance Imaging, and Bioelimination Properties of Mono- or Bisphosphonate-Anchored Dendronized Iron Oxide Nanoparticles", ChemPubSoc, vol. 82, no. 4, 1st April 2017, pages 647-659, and "Validation of a dendron concept to tune colloidal stability, MRI relaxivity and bioelimination of functional nanoparticles", Materials Chemistry B, 9th January 2015, pages 1459-1732.

Other examples are provided in EP 3 125 949 A1 and in the articles by Shi D., et al., entitled "Interfacial Behavior of Oligo(Ethylene Glycol) Dendrons Spread Alone and in Combination with a Phospholipid as Langmuir Monolayers at the Air/Water Interface", Molecules, vol. 24, no. 22, 14th November 2019, and "Microbubbles decorated with dendronized magnetic nanoparticles for biomedical imaging: effective stabilization via fluorous interactions", Belstein Journal of Nanotechnology, vol. 10, no. 10 31st October 2019, pages 2103-2115.

A first objective of the present invention is to provide a new class of dendritic molecules that makes possible the delivery and targeting of many diagnostic and/or therapeutic agents.

A second objective of the present invention is to provide a new class of dendritic molecules that can be used in phase change emulsions (PCEs) and able to (i) control the size and stabilize nanodroplets, (ii) precisely control the phase change phenomenon, (iii) obtain predetermined microbubbles sizes and size distributions, and (iv) stabilize these microbubbles.

A third objective of the present invention is also to provide a new class of dendritic molecules effective and useful in controlling the properties of nanoemulsions and microbubbles, independently of the PCE.

Finally, a fourth objective of the present invention is to provide a preparation process allowing the synthesis of this new class of dendritic molecules.

These objectives are reached by the dendritic molecules of formula (I) as described in detail thereafter and by their preparation processes.

### Description of the invention

A first object of the present invention is a dendritic molecule of formula (I) below: 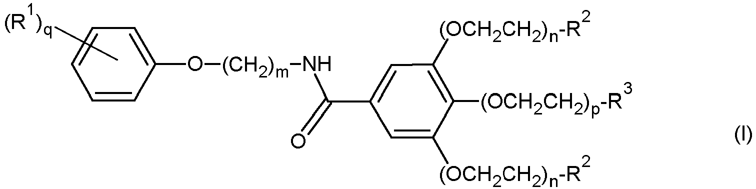 wherein :
- R¹ is a phosphonate group of the following formula (PG): 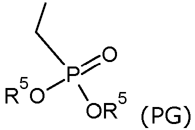 in which each of R⁵ represents a linear alkyl radical having at least 4 carbon atoms and the star represents the attachment point of said group of formula (PG) to the phenyl cycle;
- each of R² represents a linear alkyloxy radical having from 1 to 20 carbon atoms;
- R³ represents a linear alkyloxy radical having from 1 to 20 carbon atoms, a carboxyl group or a group -COO*t*Bu in which tBu means ter-butyl;
- n is an integer ranging from 1 to 16;
- p is an integer ranging from 1 to 16;
- m is an integer ranging from 1 to 4, preferably m = 1 or 2 and more preferably m = 2; and

According to a preferred embodiment of the present invention, n is an integer ranging from 4 to 6 inclusively and even more preferably n = 4.

According to another preferred embodiment of the present invention, p is an integer ranging from 4 to 10 inclusively and even more preferably p = 4 or p = 9.

According to a particular and preferred embodiment of the present invention, q = 2 and R¹ represents a phosphonate group of formula (PG) in which each of R⁵ represents an alkyl group having from 4 to 12 carbon atoms, more preferably R⁵ is an alkyl group selected among octyl, decanyl, and dodecanyl.

According to another particular and preferred embodiment of the present invention, R³ represents a methyloxy group, a carboxyl group or a group -COOtBu in which *t*Bu means *ter*-butyl.

According to a most preferred embodiment of the present invention, compounds of formula (I) according to the present invention are selected among compounds of formulae (I-A) to (I-F) whose significations of R¹ to R⁵, m, n, p, and q are given in the following Table 1:

**TABLE 1**

| **Cpds** | **R¹** | **R²** | **R³** | **R⁴** | **R⁵** | **n** | **p** | **m** | **q** |
|---|---|---|---|---|---|---|---|---|---|
| (I-A) | PG | -OCH₃ | -OCH₃ | - | -(CH₂)₇-CH₃ | 4 | 4 | 2 | 2 |
| (I-B) | PG | -OCH₃ | -OCH₃ | - | -(CH₂)₉-CH₃ | 4 | 4 | 2 | 2 |
| (I-C) | PG | -OCH₃ | -OCH₃ | - | -(CH₂)₁₁-CH₃ | 4 | 4 | 2 | 2 |
| (I-D) | PG | -OCH₃ | -COOH | - | -(CH₂)₇-CH₃ | 4 | 9 | 2 | 2 |
| (I-E) | PG | -OCH₃ | -COOH | - | -(CH₂)₉-CH₃ | 4 | 9 | 2 | 2 |
| (I-F) | PG | -OCH₃ | -COOH | - | -(CH₂)₁₁-CH₃ | 4 | 9 | 2 | 2 |

A second object of the present invention is a process for the preparation of the dendritic molecules of formula (I) in which R¹ is a phosphonate group of formula (PG), q = 2, n = p and R² and R³ are identical and represent an alkyloxy group as defined above in formula (I), said process comprising at least one step of reacting a compound of formula (II) below: 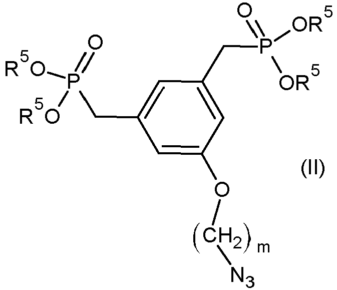 wherein R⁵ and m have the same signification as in formula (I) above, with a compound of formula (III) below: 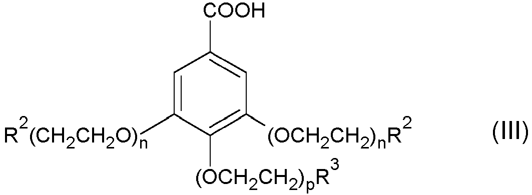 wherein n = p and have the same definition as in formula (I) above and further wherein R² and R³ are identical and represent an alkyloxy group as defined in formula (I), to lead to the corresponding compound of formula (I).

The reaction of compounds of formulae (II) and (III) can be carried out at room temperature, i.e. at a temperature ranging from 18 to 25°C, by mixing a solution of a compound of formula (II) in an appropriate solvent such as for example ethyl acetate, in the presence of a catalyst such as palladium/C, with a solution of a compound of formula (III) in an appropriate solvent such as for example dichloromethane in the presence of oxalyl chloride, dimethylformamide and N,N-diisopropylethylamine. The resulting compound of formula (I) can then be recovered and purified according to the usual practice known from one skilled in the art.

Compounds of formula (III) may be prepared according to a process comprising at least the following steps:
- reacting an alcohol of formula R⁶-(OCH₂CH₂)ₙOH (IV) in which R⁶ represents a linear alkyloxy radical having from 1 to 20 carbon atoms with tosyl chloride to obtain a compound of formula (VI) below: 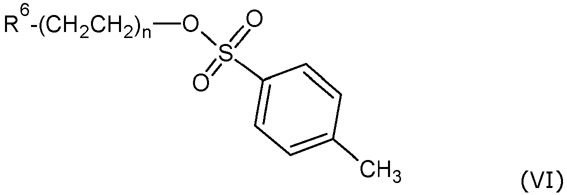 wherein R⁶ has the same meaning as in formula (IV);
- reacting said compound of formula (VI) with methyl gallate to obtain a compound of formula (VIII) below: 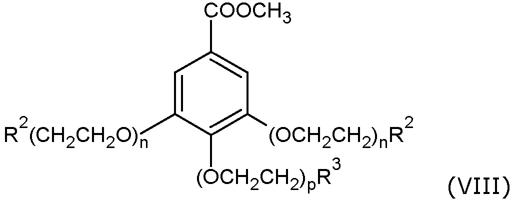 wherein n, p, R² and R³ have the same meaning as in formula (III); and
- unprotecting the carboxyl function of compound of formula (VIII) thus obtained to lead to the corresponding compound of formula (III).

The process for the preparation of compounds of formula (III) can be represented by the following Scheme 1:

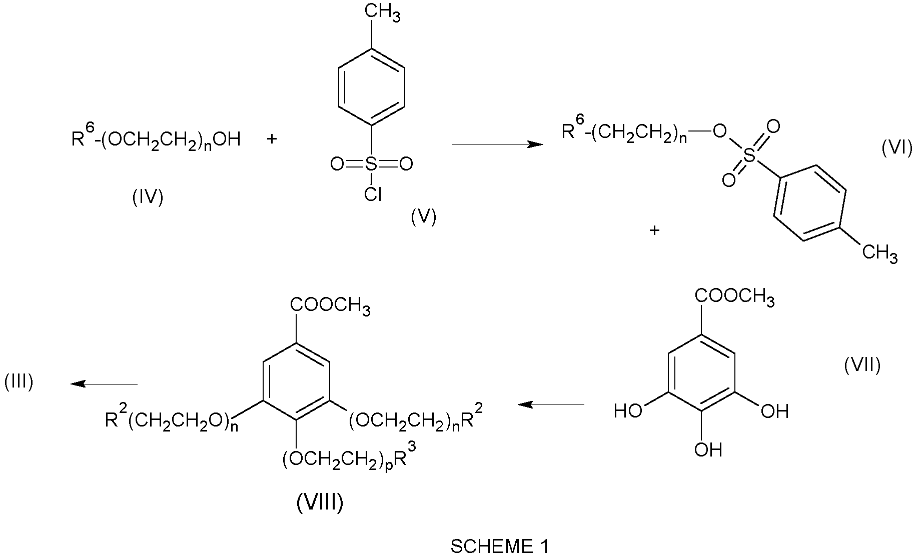

According to the process represented on Scheme 1, a solution of a compound of formula (IV) in which R⁶ has the same meaning as R² and R³ in the compounds of formula (III) above, (R² and R³ being identical) in an appropriate solvent such as for example dichloromethane in the presence of an amine such as for example triethylamine is contacted with a compound of formula (V), at room temperature under mixing until a compound of formula (VI) is obtained wherein R⁶ has the same meaning as in formula (IV) above, (R² and R³ being identical). The compound of formula (VI) is then reacted with a solution of compound of formula (VII) (methylgallate) in an appropriate solvent such as for example acetone, in the presence of potassium carbonate and potassium iodide and heated to reflux under mixing for about 8 to 16 hours to obtain a compound of formula (VIII) in which R² is identical to R³ and is an alkyl group as defined above in formula (I). The carboxyl group of compound of formula (VIII) is then unprotected by reacting said compound of formula (VIII) dissolved in an appropriate solvent such a lower alcohol, i.e. methanol or a mixture of a lower alcohol with water, in particular a mixture of methanol and water, in the presence of an alkalinizing agent such as for example sodium hydroxide, at room temperature, to lead to the corresponding compound of formula (III).

Compounds of formula (II) may be prepared according to a process comprising at least the following steps:
- reacting an alcohol of formula R⁵-OH (IX) wherein R⁵, has the same meaning as in formula (I) with trimethylphosphite (compound of formula (X)) to obtain a compound of formula (XI) below: 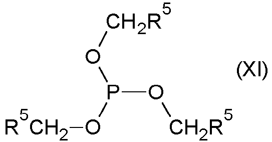 wherein R⁵, has the same meaning as in formula (I);
- reacting the compound of formula XI thus obtained with 3,5-bis(bromomethyl)phenol (compound of formula (XII)) to obtain a compound of formula (XIII) below: 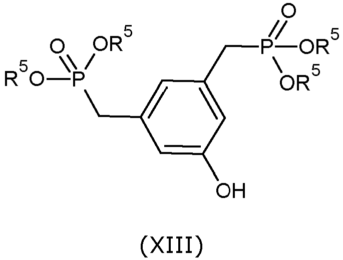 wherein R⁵, has the same meaning as in formula (I); and
- reacting the compound of formula (XIII) thus obtained with a compound of formula (XIV) below: 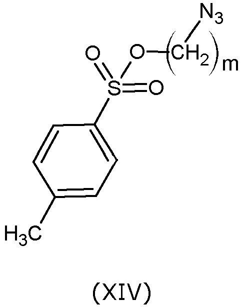
in which m has the same meaning as in formula (I) to obtain the corresponding compound of formula (II).

The process for the preparation of compounds of formula (II) can be represented by the following Scheme 2:

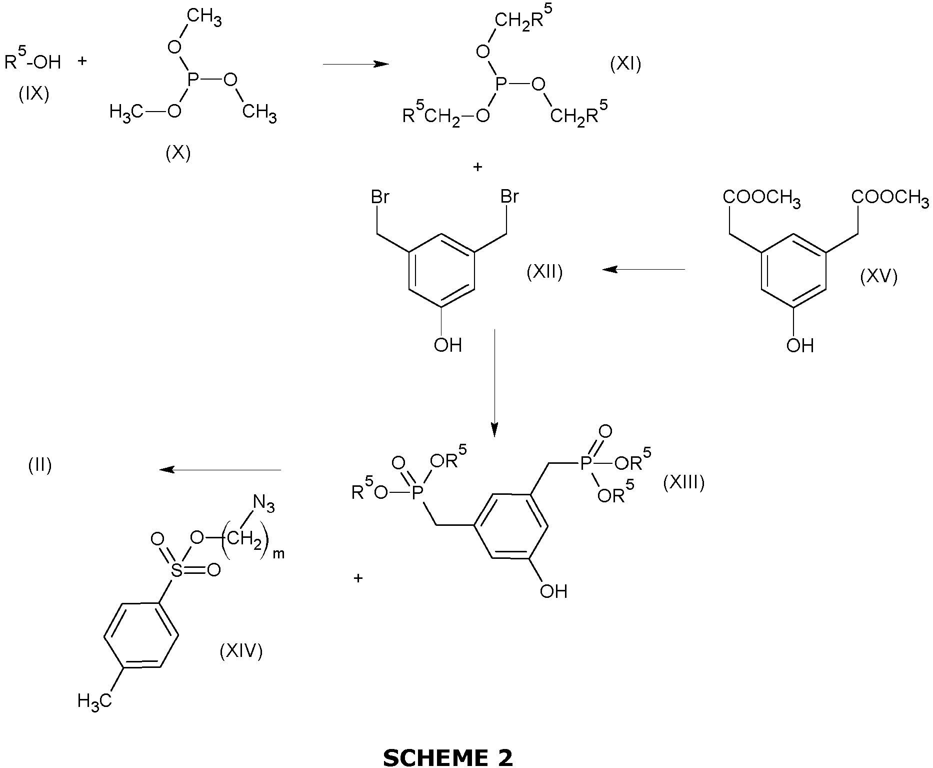

According to the process represented on Scheme 2, a compound of formula (X) (trimethylphosphite) is added to an alcohol of formula (IX) in which R⁵ has the same meaning as in formula (I) above, said alcohol of formula (IX) having previously been heated at a temperature of 30 to 75°C. The resulting mixture is then heated to a temperature of 130 to 230°C under argon atmosphere during 5 to 16 hours, to lead to compound of formula (XI) in which R⁵ has the same meaning as in formula (I) above. Compound (XI) may be separated from the remaining alcohol of formula (IX) for example by distillation. Compound of formula (XI) is then contacted with a compound of formula (XII) under stirring at a temperature of about 110 to 150°C for a period of time ranging from 8 to 16 hours to lead to compound of formula (XIII) in which R⁵ has the same meaning as in formula (I) above. A compound of formula (XIV) in which m has the same meaning as in formula (I) is added to a solution of the compound of formula (XIII) in an appropriate solvent such as for example toluene, said solution comprising an alkalinizing agent such as for example potassium hydroxide and potassium iodide and being previously heated at a temperature of 60 to 90°C. The resulting mixture is maintained at a temperature of 60 to 90°C and stirred for 8 to 16 hours to lead to a compound of formula (II) which can be recovered and purified by the usual techniques well known from one skilled in the art.

Compound of formula (XIV) can be previously prepared according to the process represented by the following Scheme 3:

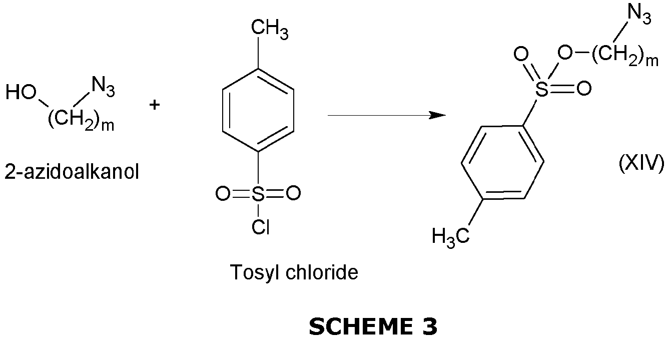

According to the process represented on Scheme 3, a solution of a 2-azidoalkanol in which m has the same meaning as in formula (I) in an appropriate solvent such as for example dichloromethane, is reacted with tosyl chloride in the presence of trimethylamine at room temperature for 8 to 16 hours to lead to a compound of formula (XIV) in which m has the same meaning as in formula (I).

A third object of the present invention is a process for the preparation of the dendritic molecules of formula (I) in which R¹ is a phosphonate group (PG), q = 2, n and p are identical or different and in which R² is an alkyloxy group as defined above in formula (I) and R³ is a carboxyl group or a group -COOtBu, said process comprising at least the following steps:
- reacting methyl gallate (compound of formula (VII) as defined above) with benzyl bromide to obtain a compound of formula (XV) below: 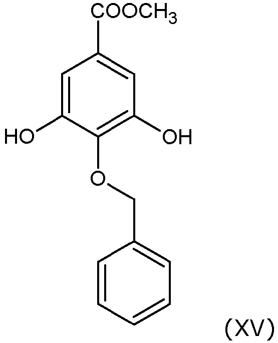
- independently reacting an alcohol of formula R²-(CH₂CH₂)ₙOH (IV') wherein R² and n have the same meaning as in formula (I) above with tosyl chloride (compound of formula (V) as defined above) to obtain a compound of formula (VI') below: 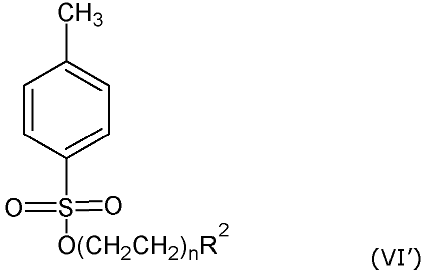 wherein R² and n have the same meaning as in formula (I) above;
- reacting the compound of formula (XV) and the compound of formula (VI') thus obtained to obtain a compound of formula (XVI) below: 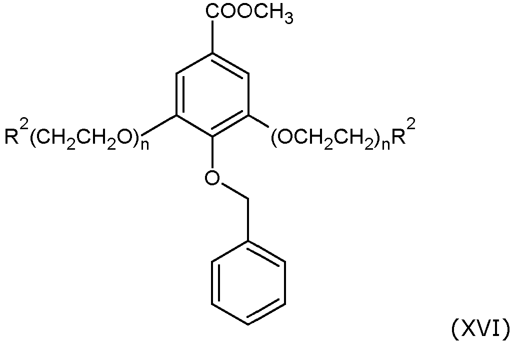 wherein R² and n have the same meaning as in formula (I) above;
- unprotecting the carboxylic function of the compound of formula (XVI) thus obtained to lead to the compound of formula (XVII) below: 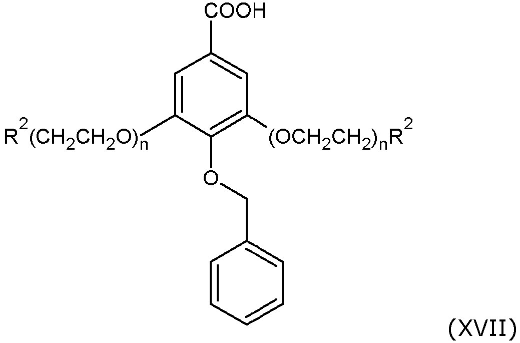 wherein R² and n have the same meaning as in formula (I) as defined above;
- reacting the compound of formula (XVII) thus obtained with a compound of formula (II) as defined above to obtain a compound of formula (XVIII) below: 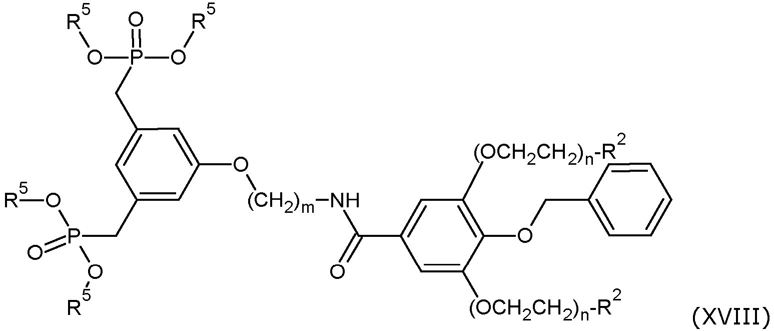 wherein R⁵, R², m and n have the same meaning as in formula (I) above;
- hydrolyzing the benzyl group of the compound of formula (XVIII) thus obtained to obtain a compound of formula (XIX) below: 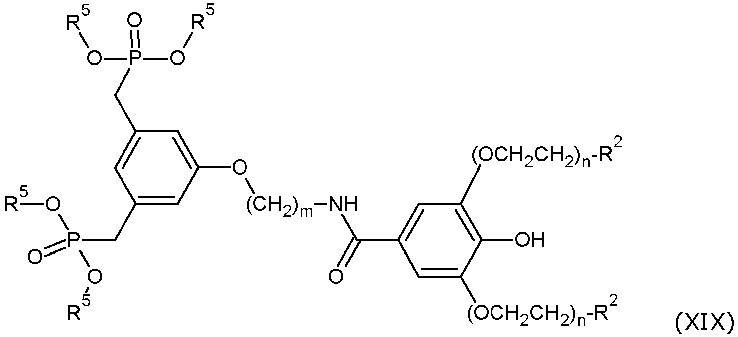 wherein R⁵, R², m and n have the same meaning as in formula (I) above;
- reacting the compound of formula (XIX) thus obtained with a compound of formula (XX) below: 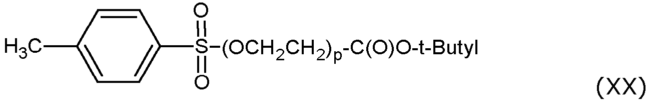 wherein p has the same meaning as in formula (I) and has a value which is identical or different to the value of n of the compound in formula (XIX) as defined above, to obtain a compound of formula (XXI) below: 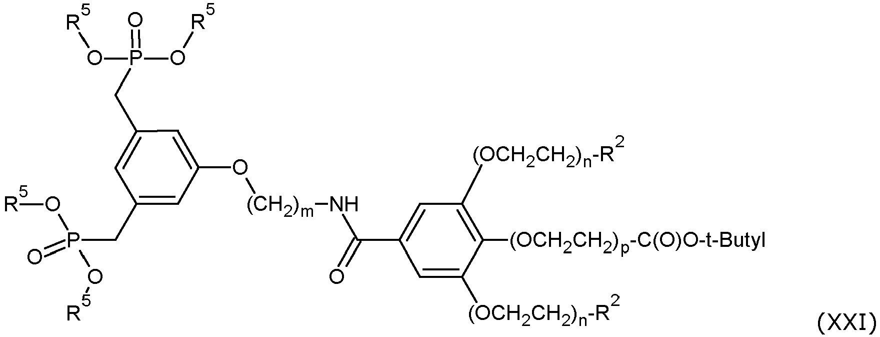 wherein R⁵, R² m, n and p have the same meaning as in formula (I) above and p has the same meaning as in formula (I) and has a value which is identical or different to the value of n in the compound of formula (XIX) as defined above and which corresponds to a particular compound of formula (I) in which R³ is a group -COO*t*Bu ; and optionally
- unprotecting the carboxylic function of compound of formula (XXI) thus obtained to lead to the corresponding dendritic molecule of formula (I) in which R³ is a group -COOH.

The process for the preparation of the dendritic molecules of formula (I) in which n and p are identical or different and in which R² is an alkyloxy group as defined above in formula (I) and R³ is a carboxyl group can be represented by the following Scheme 4:

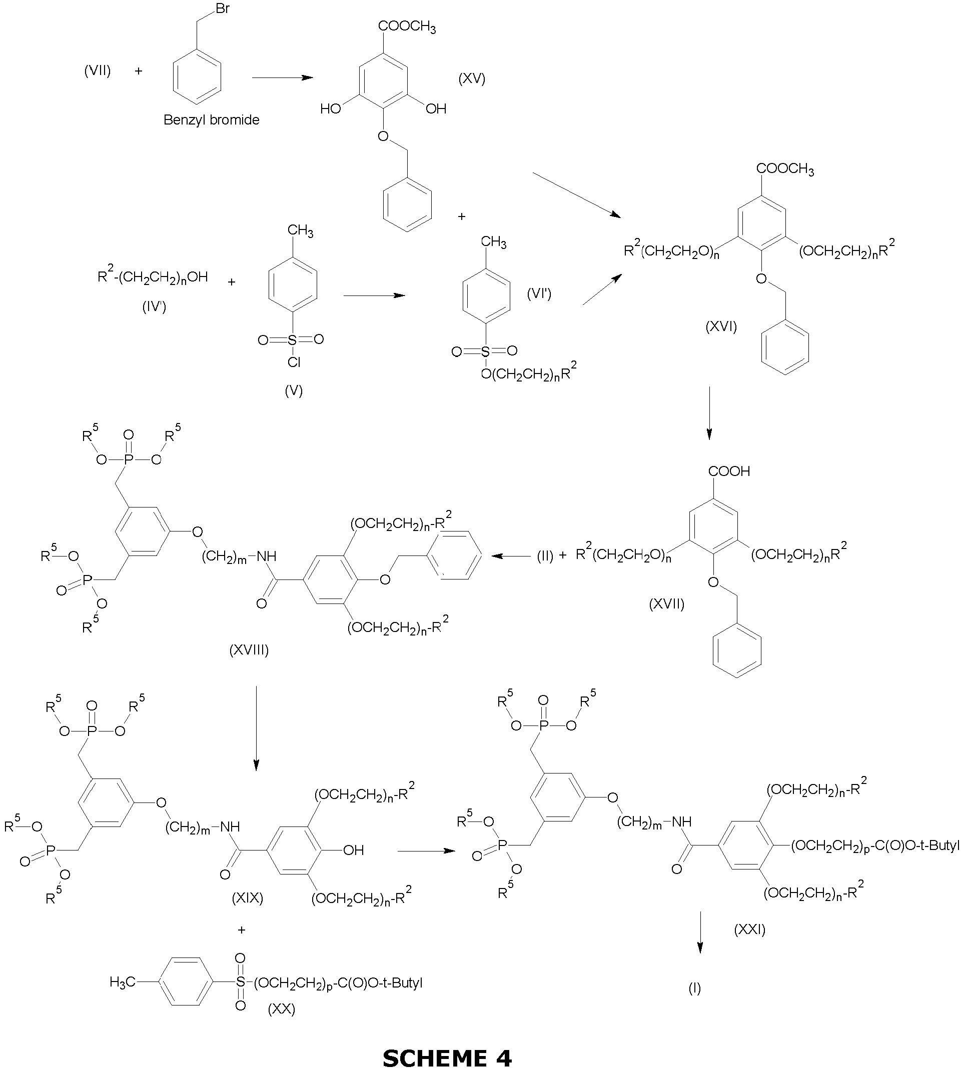

According to the process represented on Scheme 4, a compound of formula (VII) as defined above in Scheme 1 in solution in an appropriate solvent such as for example dimethylformamide is reacted with benzyl bromide in the presence of potassium hydrogen carbonate and potassium iodide at room temperature for 8 to 24 hours to lead to compound of formula (XV). Independently, a solution of a compound of formula (IV') in which R² as the same meaning as in formula (I) above, in an appropriate solvent such as for example dichloromethane in the presence of an amine such as for example trimethylamine, is contacted with a compound of formula (V), at room temperature under mixing until a compound of formula (VI') wherein R² as the same meaning as in formula (I) above is obtained. A solution of the compound of formula (XV) in an appropriate solvent such as for example acetone is then reacted with compound of formula (VI') thus obtained in the presence of an alkalinizing agent such as for example potassium carbonate, and potassium iodide. The resulting mixture is then heated at reflux for 8 to 24 hours to lead to the corresponding compound of formula (XVI) wherein R² and n have the same meaning as in the compound of formula (VI'). The carboxyl group of compound of formula (XVI) thus obtained is then unprotected by reacting said compound of formula (XVI) dissolved in an appropriate solvent such a lower alcohol, i.e. methanol or a mixture of a lower alcohol with water, in particular a mixture of methanol and water, in the presence of an alkalinizing agent such as for example sodium hydroxide, at room temperature, to lead to the corresponding compound of formula (XVII) wherein R² and n have the same meaning as in the compound of formula (XVI). The reaction of compounds of formula (II) as defined above with reference to the process represented in Scheme 2 and in which the radicals R⁵ and m have the same meaning as in formula (I) with the compound of formula (XVII) thus obtained can be carried out at room temperature, by mixing a solution of said compound of formula (II) in an appropriate solvent such as for example ethyl acetate, in the presence of a catalyst such as palladium/C, with a solution of a compound of formula (XVII) in an appropriate solvent such as for example dichloromethane in the presence of oxalyl chloride, dimethylformamide and then of N,N-diisopropylethylamine to lead to the corresponding compound of formula (XVIII) in which R⁵, R², m and n have the same meaning as in formula (I). A solution of the resulting compound of formula (XVIII) in an appropriate solvent such as for example ethyl acetate comprising a catalyst such as for example palladium/C is then purged with a hydrogen atmosphere in stirred at room temperature for 5 to 24 hours to lead to the corresponding compound of formula (XIX) in which R⁵, R², m and n have the same meaning as in formula (I). A solution of the compound of formula (XIX) thus obtained in an appropriate solvent such as for example acetone is then contacted with a compound of formula (XX) in which p has the same meaning as in formula (I) above, the value of p being equal of different to the value of n in the compound of formula (XIX), in the presence of potassium carbonate and potassium iodide. The resulting mixture is then heated at reflux for 8 to 24 hours to lead to the corresponding compound of formula (XXI) in which R⁵, R², m, n and p are as defined previously. Compounds of formula (XXI) correspond to compounds of formula (I) in which R³ is a group -COO*t*Bu. The resulting compound of formula (XXI) can then be unprotected by addition of a strong acid, such as for example trifluoroacetic acid, into a solution of said compound of formula (XXI) in an appropriate solvent such as for example dichloromethane, and stirring at room temperature for 1 to 2 hours to lead to the expected corresponding compound of formula (I). Compounds of formula (XXI) and of formula (I) can be recovered and purified according to the usual practice known from one skilled in the art.

Compounds of formula (XX) can be prepared according to a process comprising the step of reacting tosyl chloride with a compound of formula (XXII): OHCH₂CH₂-(OCH₂CH₂)ₚ₋₁-C(O)O-*t*-Butyl wherein p has the same meaning as in formula (I) above.

This process can be represented by the following Scheme 5:

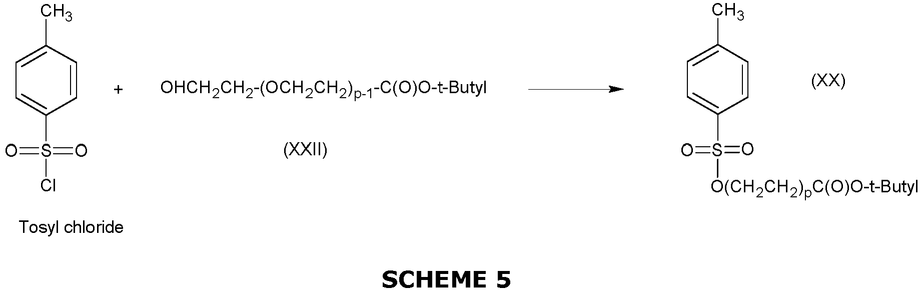

According to the process represented by Scheme 5, tosyl chloride is reacted with a solution of a compound of formula (XXII) in which p has the same meaning as in the compound of formula (I) and wherein p can have the same value than n or a different value than n, in an appropriate solvent such as for example dichloromethane, in the presence of an amine such as for example trimethylamine at room temperature for 8 to 24 hours. Compound of formula (XXII) thus obtained can then be recovered and purified by the technics well known by one skilled in the art.

The dendritic molecules of formula (I) according to the present invention are useful as drug carriers, in particular as delivery vehicles of drugs, in particular of nonsteroidal anti-inflammatory drugs (NSAIDs), anticancer drugs, fragment antibodies, nanobodies, and other drugs such as simvastatin, famotidine, or quinolones.

Therefore, another object of the present invention is the use of a dendritic molecule of formula (I) as defined according to the first object of the present invention, as a drug carrier.

In particular, the dendritic molecule of formula (I) in which R³ is a carboxyl group are particularly preferred for a use as a drug carrier, since they can be easily functionalized with a ligand or an active principle.

The dendritic molecules of formula (I) according to the present invention are useful as contrast agents for medical imaging or diagnosis platforms after functionalization with at least one imaging agent such as for example biocompatible fluorescent dyes, traditional small molecular contrast agents, or metal ion/chelator complexes or with at least one metallic or metallic oxide nanoparticle. In particular, dendritic molecules of formula (I) can be grafted to metallic oxide nanoparticles and used as medical imaging tool, in particular optical imaging tool or magnetic imaging tool, more particularly magnetic resonance imaging contrast agent, or magnetic particle imaging tracer, or as a hyperthermia and/or radiosensitizing agent for the treatment of tumors or other pathological tissues.

Therefore, another object of the present invention is a dendritic molecule of formula (I) as defined according to the first object of the present invention in combination with at least one imaging agent, for its use as contrast agent for medical imaging or diagnosis platforms.

The dendritic molecules of formula (I) according to the invention are also particularly useful in phase change emulsions (PCEs) to (i) control the size and stabilize nanodroplets, (ii) precisely control the phase change phenomenon, (iii) obtain predetermined microbubbles sizes and size distributions, and (iv) stabilize these microbubbles.

The dendritic molecules of formula (I) according to the invention are also finally useful in controlling the properties of nanoemulsions and microbubbles, independently of the PCE.

Other advantages and embodiments of the present invention are given by the following examples.

### EXAMPLES

### EXAMPLE 1: Synthesis of a dendritic molecule of formula (I-A) according to the invention

In this example, a dendritic molecule of the following formula (**I-A**) was prepared:

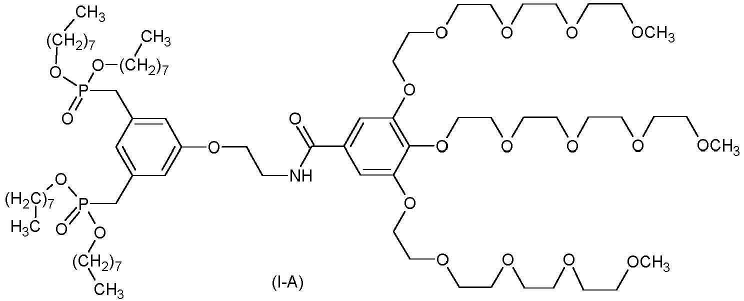

### 1.1 Step 1 - Preparation of 2,5,8,11-tetraoxatridecan-13-yl 4-methylbenzenesulfonate (compound 1)

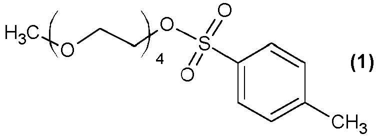

30.1 mmol (1 Eq.) of tetraethyleneglycol monomethyl ether were dissolved in 170.0 mL of dichloromethane at room temperature, then 5 mL of triethylamine (Et₃N) (36.2 mmol, 1.2 Eq.) were added and the reaction stirred for 10 min. 22.3 g (36.2 mmol, 1.2 Eq.) of tosyl chloride (solid) were slowly added. The resulting mixture was stirred at room temperature during the night.

A thin layer chromatography (TLC) analysis (stain phosphomolybdic acid (PMA) solution) showed the full consumption of PEG-OMe. The mixture was filtered on celite to remove the salts. The solvent was then evaporated under reduced pressure.

Purification by flash chromatography (100% dichloromethane (DCM) then 100% ethyl acetate (AcOEt)) afforded a clear liquid (10.1 g, 93%).

### Analysis:

¹H NMR (400 MHz, MeOD) δ 7.85 (d, J = 8.4 Hz, 2H), 7.50 (dd, J = 8.6, 0.8 Hz, 2H), 4.23 - 4.14 (m, 3H), 3.74 - 3.68 (m, 3H), 3.68 - 3.64 (m, 7H), 3.62 - 3.55 (m, 7H), 3.40 (s, 3H), 2.51 (s, 3H).

### 1.2 Step 2- Preparation of compound (2)

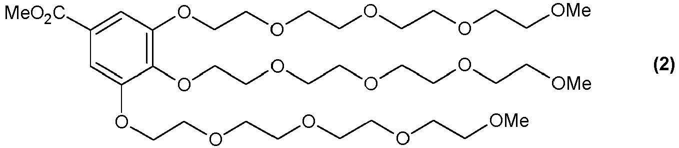

To a solution 8.8 mmol (1.0 Eq) of methyl gallate in acetone (60 mL) were added 18.1 mmol (3.2 Eq.) of potassium carbonate (K₂CO₃), 0.6 mmol (0.1 Eq.) of potassium iodide (KI) and 18.9 mmol (3.3 Eq.) of compound (1) as preparation in step 1 above. The resulting solution was heated to reflux for 42 hours.

The reaction mixture was cooled to room temperature, the solvent was removed, solid were suspended in dichloromethane (CH₂Cl₂), filtered over Celite, washed with an aqueous solution of sodium thiosulfate (Na₂S₂O₃) 2N and brine, dried over sodium sulfate (Na₂SO₄), filtered and concentrated under reduced pressure. Purification by flash chromatography (CH₂Cl₂/MeOH 98/2 to 96/4 to 9/1) gave 3.9g (89%) of a yellowish oil.

### Analysis:

¹H NMR (400 MHz, Chloroform-d) δ 7.46 (d, 2H: Ar-*H*), 4.15 (dd, *J* = 5.6, 4.2 Hz, 6H: -O-C*H₂*-R), 3.86 (s, 3H: C*H₃*-O-C=O-R), 3.85 - 3.81 (m, 6H: -O-CH₂-C*H₂*-R), 3.70 - 3.66 (m, 6H: -O-C*H₂*-R), 3.63 - 3.56 (m, 24H: -O-C*H₂*-R), 3.51 - 3.47 (m, 6H: -O-C*H₂*-R), 3.33 (s, 9H: C*H₃*-O-).

### 1.3 Step 3 - Preparation of compound (3)

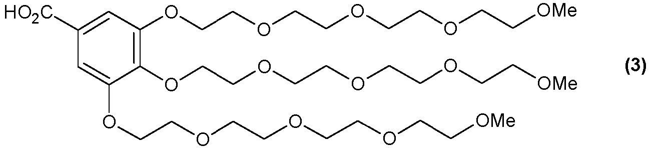

To a solution of 6.0 g (6.9 mmol - 1.0 Eq.) of compound **(2)** in methanol (MeOH) were successively added 1.1 g (34.5 mmol - 5.0 Eq.) of sodium hydroxide (NaOH) and 15.0 mL of distilled water. The yellow solution was stirred at room temperature (RT) overnight.

TLC analysis showed the consumption of compound **(2).** The solvents were removed by rotavap, then the crude product dissolved in CH₂Cl₂. HCl 2N (20.0 mL) was added and the stirring was continued for 15 min. The organic product was collected with CH₂Cl₂, the aqueous layer was washed with CH₂Cl₂ (five times), the combined organic layer was washed with brine, dried over Na₂SO₄, filtered and concentrated under reduced pressure to yield to compound (3) (5.1 g, 6.8 mmol, 99%) as yellow oil.

### Analysis:

¹H NMR (400 MHz, Chloroform-d) δ 7.46 (d, 2H : Ar-*H*), 4.15 (dd, *J* = 5.6, 4.2 Hz, 6H: -O-C*H₂*-R), 3.85 - 3.81 (m, 6H : -O-CH₂-C*H₂*-R), 3.70 - 3.66 (m, 6H : -O-C*H₂*-R), 3.63 - 3.56 (m, 24H : -O-C*H₂*-R), 3.51 - 3.47 (m, 6H : -O-C*H₂*-R), 3.33 (s, 9H : C*H₃*-O-).

### 1.4 Step 4 - Preparation of 3,5-bis(bromomethyl)phenol (compound (4))

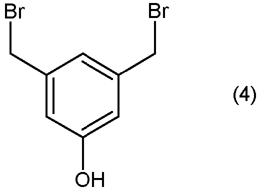

72 mL of lithium aluminum hydride 1M (LiAlH₄) (72 mmol - 1.8 Eq.) were dissolved in 150 mL of tetrahydrofuran (THF) at 0°C. Then 8.40 g (40 mmol - 1 Eq.) of dimethyl 5-hydroxyisophthalate were carefully added. The resulting solution was stirred at RT for 5 hours, then EtOAc (30.0 mL) and an aqueous solution of H₂SO₄ 10% (60.0 mL) were carefully added at 0°C. The stirring was continued overnight. An additional aqueous solution of H₂SO₄ 10% (60.0 mL) was added. The stirring was continued at RT for another 24h.

Once the aluminum salt was entirely dissolved, the aqueous layer was washed with EtOAc (at least 5 times). The combined organic layer was dried over Na₂SO₄, filtered and concentrated under reduced pressure to yield 8.3831 g of orange oil. The crude benzylic alcohol was then dissolved in 100 mL of acetic acid (AcOH) before 36 mL of hydrobromic acid (HBr 33% w/w in AcOH) (200 mmol - 5.0 Eq.) was carefully added at 0°C. The resulting mixture was stirred at RT for 2 days.

TLC analysis showed the full consumption of bis-benzylic alcohol, which is the intermediate formed but not isolated following the reduction of dimethyl 5-hydroxyisophthalate by LiAlH₄. The organic product was washed with brine (1 time), the aqueous layer was washed with EtOAc (at least five times), the combined organic layer was dried over Na₂SO₄, filtered and concentrated under reduced pressure. Purification by flash chromatography (CH₂Cl₂/EtOAc 1/0 to 96/4 to 9/1) afforded a yellowish solid. Recrystallization with a mixture of petroleum ether/diethyl oxide (EtP/Et₂O) afforded compound (4) (10.40 g, 37.4 mmol, 93.6%) as white solid.

### Analysis:

¹H NMR (400 MHz, Chloroform-d) δ 6.99 (s, 1H: Ar-*H*), 6.81 (d, *J* = 1.43 Hz, 2H: Ar-*H*), 4.41 (s, 4H: -C*H₂*-Ar).

### 1.5. Step 5 - Preparation of compound (5)

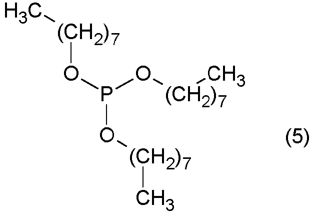

In a 100 mL two-necked flask connected to a Dean-Stark, 33.9 mL of octanol (107.25 mmol - 6 Eq.) were added, then the reaction setup was stirred and passed under a flow of argon for 10 min.

After that, the liquid was heated to 75°C and the flow replaced by 2 balloons of argon. 4.23 mL of trimethylphosphite (35.8 mmol - 1 Eq.) were added slowly from the syringe and then the setup was heated at 220 °C for 6 h.

Then the setup was cooled to ambient temperature, and the Dean-Stark containing the methanol formed during the reaction removed. An orange oil was obtained.

Distillation under reduced pressure was used to separate the remaining octanol from the compound **(5)** formed, at 160°C.

### 9.205 g of orange oil were recovered (yield = 61%)

### Analysis:

¹H-NMR (400MHz, Chloroform-d): δ 3.77 (q, J = 6.9 Hz, 6H, O-C*H₂*-R), 1.61 - 1.57 (m, 6H, O-C*H₂*-CH₂-R), 1.28 - 1.25 (m, 30H, C*H₂*), 0.86 (t, J = 6.7 Hz, 9H, C*H₃*-R).

### 31P-NMR (400MHz, CDCl₃): δ 139.15

### 1.6. Step 6 - Preparation of compound (6)

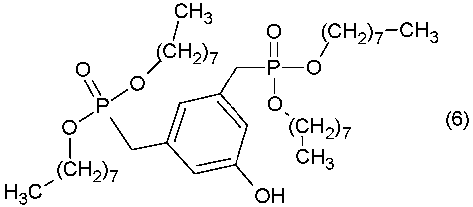

152 mg (0.543 mmol - 1 Eq.) of compound **(4)** and 916 mg (2.188 mmol - 4.0 Eq.) of compound **(5)** were added to a 50 mL flask. The reaction was stirred and heated to 140°C. overnight. A yellow-orange oil was obtained and a TLC was carried out (Petroleum ether / EtOAc 1/1):

The next day, after having cooled the solution, a Petroleum Ether / EtOAc chromatographic column was produced (1/1 to 2/3).

248 mg of compound **(6)** were recovered (yield = 64%).

### Analysis:

¹H NMR (400 MHz, CDCl₃) δ 6.81 (s, 2H, Ar-*H*), 6.64 (s, 1H, Ar-*H*), 4.02 - 3.77 (m, 8H, P-C*H*₂), 3.28 - 2.81 (m, 4H, Ar-C*H*₂-P), 1.58 (m, 8H, P-CH₂-C*H*₂), 1.42 - 1.14 (m, 44H, C-C*H*₂), 0.95 - 0.77 (t, *J* = 7.1Hz, 12H, C-C*H*₃).

³¹P NMR (162 MHz, CDCl₃) δ 26.51.

### 1.7. Step 7 - Preparation of linker 2 (L2)

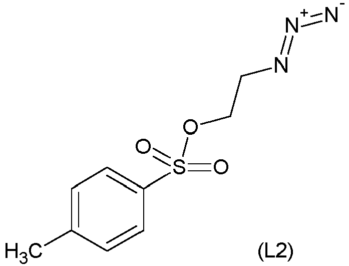

### 1.7.1. Step 7.1 - Preparation of ligker 1 (L1)

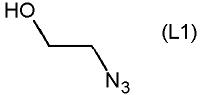

10.0 g (77.6 mmol - 1 Eq.) of 2-bromoethanol (97%) were dissolved in 12 mL of water, then at RT 6.12 g (93.2 mmol - 1.2 Eq.) of sodium azide (NaN₃) were added. The mixture was heated at 80°C for the night. Monitoring was made by TLC (DCM/MeOH, 95:5, SM Rf=0, EP Rf= 0,5).

After the night TLC showed just a few traces of the starting material, the mixture was stopped, then 10 mL of DCM was added. Phases were separated. Aqueous was extracted with 20 mL of DCM and NaCl solid was added at each extraction. The organic phase was directly engaged in the next step, without evaporation or further purification.

### 1.7.2. Step 7. - Preparation of linker 2 (L2)

To 6.76 g (77.6 mmol - 1 Eq.) of Linker 1 in DCM, were added 16.4 mL (116.0 mmol - 1.5 Eq.) of Et₃N slowly, the mixture was stirred 10 min, then 17.9 g (93.2 mmol - 1.2 Eq.) of tosyl chloride (TsCl) were added slowly.

After the night TLC (petroleum ether, AcOEt 7:3, KMnO₄) showed traces of the starting material. The mixture was concentrated, TsCl salts were precipitated in AcOEt and filtered over celite pad, washed 3 times with AcOEt, to give the desired linker 2 (22 g, brown oil).

It was then purified by Flash chromatography, Interchim-220g-30, liquid deposit in EtP/AcOEt (9:1), Eluent: 15 min EtP/AcOEt (9:1), 10 min EtP/AcOEt (8:2) with isocratic, and 10min EtP/AcOEt (7:3).
12.13 g of Linker 2 as a colourless oil was recovered.
¹H NMR (400 MHz, MeOD) δ 7.84 (d, *J* = 8.4 Hz, 2H), 7.49 (d, *J* = 7.8 Hz, 2H), 4.25 - 4.12 (m, 2H), 3.54 - 3.40 (m, 3H), 2.49 (s, 3H).

### 1.8. Step 8 - Preparation of compound (7)

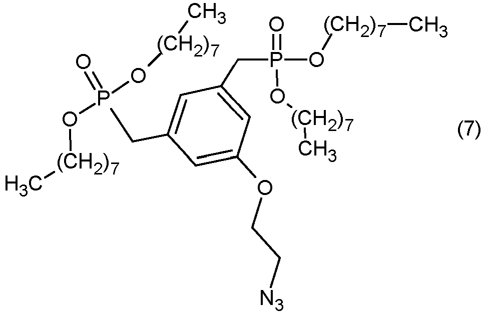

In a 25 mL flask were added 297.0 mg (1.23 mmol - 1.8 Eq.) of compound **(6),** 57.2 mg (1.02 mmol - 1.5 Eq.) of potassium hydroxide (KOH) and 11.3 mg (0.068 mmol - 0.1 Eq.) of KI in 15 mL of toluene. The solution was stirred for 20 min and heated to 60°C. to dissolve the reagents. 500.0 mg (0.684 mmol - 1.0 Eq.) of linker 2 (as prepared above in step 1.7. of example 1) was added and the reaction was left to stir overnight.

The flask was cooled to RT, then the toluene was evaporated off under reduced pressure. The crude as then dissolved in DCM, and filtered through Celite. A chromatographic column was carried out DCM / MeOH (1/0 to 98/2 to 96/4). 530 mg of compound **(7)** (97%) were thus recovered.

### Analysis:

¹H NMR (400 MHz, MeOD) δ 6.90 - 6.82 (m, 3H, Ar-H), 4.17 (t, *J* = 4.9 Hz, 2H, O-C*H₂*-CH₂-N₃), 3.98 (m, 8H, P-O-C*H₂*), 3.59 (t, *J* = 4.8 Hz, 2H, O-CH₂-C*H₂*-N₃), 3.27 - 3.16 (BX, *J* = 21.9 Hz, 4H, Ar-C*H₂*-P), 1.67 - 1.55 (m, 8H, P-O-CH₂-C*H*₂), 1.41 - 1.26 (m, 40H, C-C*H*₂), 0.95 - 0.85 (t, *J* = 6.5Hz, 12H, C-C*H*₃).

¹³C NMR (101 MHz, MeOD) δ 160.00, 134.57, 134.51, 134.45, 125.49, 115.95 (C-*Ar*), 68.46, 67.76, 67.73, 67.69, 51.32, 34.29, 33.01, 32.92, 31.67, 31.64, 31.61, 30.41, 30.29, 26.70, 23.75, 14.47 (C-*C*H₃).

³¹P NMR (162 MHz, MeOD) δ 27.11.

### 1.9. Step 9 - Preparation of a dendritic molecule of formula (I-A)

To a solution of 350.0 mg (0.434 mmol - 1.1 Eq.) of compound **(7)** in 15 mL of EtOAc were added 120 mg (0.1 Eq.) of Pd/C 10% as catalyst. The resulting mixture was purged with an atmosphere of hydrogen (five times), then vigorously stirred at RT for 5h. In a separate flask, 300 mg (0.398 mmol - 1 Eq.) of compound **(3)** were dissolved in 10.0 mL of CH₂Cl₂ before 0.22 mL (1.2 mmol - 3.0 Eq.) of oxalyl chloride (COCl)₂ and 4 drops of dimethyl formamide (DMF) were added. The orange solution was stirred at RT for 5h. When TLC confirmed the full consumption of compound **(7),** the catalyst was filtered over Celite and the crude product was concentrated under reduced pressure at RT. The acyl chloride was simultaneously concentrated under reduced pressure, dissolved in 10 mL of CH₂Cl₂ then the crude primary amine (intermediate product formed but not purified) and 0.16 mL (0.916 mmol - 2.3 Eq.) of N,N-Diisopropylethylamine (DIPEA) were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure to yield 7.17 g orange oil. Purification by flash chromatography (100% EtOAc then CH₂Cl₂/EtOAc/MeOH 80/12/8 to 6/0/4) were conducted to separate the pure and impure fractions, to afford the dendritic molecule of formula (I-A) (487.2 mg, 81%) as a colorless oil.

### Analysis:

¹H NMR (400 MHz, MeOD) δ 7.23 (s, 2H), 6.85 (m Hz, 3H), 4.22 (m, 4H), 4.17 (t, *J* = 5.7 Hz, 2H), 3.97 (m, 8H), 3.91 - 3.84 (t, *J* = 4.6 Hz, 2H), 3.83 - 3.76 (m, 2H), 3.78 - 3.68 (m, 6H), 3.69 - 3.57 (m, 20H), 3.56 - 3.48 (m, 6H), 3.33 (m, 9H), 3.24 - 3.14 (AB_{X}, *J* = 22 Hz, 4H), 1.60 (m, 8H), 1.29 (m, 38H), 0.90 (t, *J* = 6.8 Hz, 12H).

³¹P NMR (162 MHz, CDCl₃) δ 25.88.

### EXAMPLE 2: Synthesis of a dendritic molecule of formula (I-B) according to the invention

In this example, a dendritic molecule of the following formula **(I-B)** was prepared:

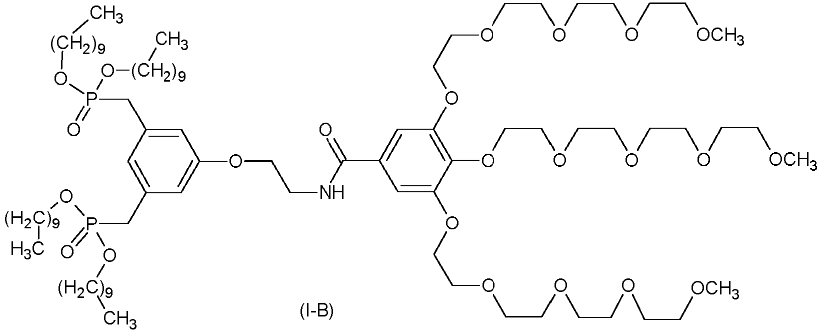

### 2.1. Step 1 - Preparation of compound (8)

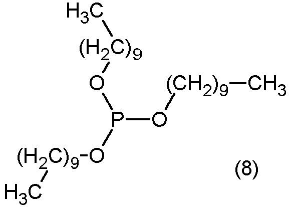

In a 100mL two-necked flask connected to a Dean-Stark, 91.4 mL (469.0 mmol - 6.0 Eq.) of decanol was added, then the reaction setup was stirred and passed under a flow of argon for 10 min.

After that, the liquid was heated to 75°C and the flow replaced by 2 balloons of argon. 9.51 mL (78.2 mmol - 1.0 Eq.) of trimethylphosphite were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining decanol from the compound (8) thus formed, at 160°C.

36.2 g of compound (8) as a colourless oil were recovered (yield = 92%).

### Analysis:

¹H NMR (400 MHz, CDCl₃) δ 3.77 (m, 6H), 1.64 - 1.55 (m, 6H), 1.41 - 1.18 (m, 44 H), 0.87 (t, *J* = 6.8 Hz, 9H).

¹³C NMR (101 MHz, CDCl₃) δ 62.48, 62.37, 32.04, 32.02, 31.33, 31.28, 29.76, 29.75, 29.71, 29.67, 29.64, 29.47, 29.43, 26.00, 22.82, 14.23.

31P-NMR (400MHz, CDCl₃): δ 139.19.

### 2.2. Step 2 - Preparation of compound (9)

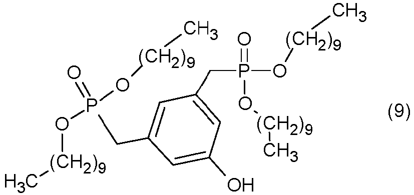

2.0 g (7.14 mmol - 1.0 Eq.) of compound **(4)** as prepared in step 1.4 of Example 1 above and 14.4 g (28.6 mmol - 4.0 Eq.) of compound **(8)** were added to a 100 mL flask. The reaction was stirred and heated to 140°C. overnight. A yellow-orange oil was obtained; a TLC was carried out (Petroleum ether / EtOAc 1/1).

The next day, after having cooled the solution, a Petroleum Ether / EtOAc chromatographic column was produced (1/1 to 2/3).

4.9 g of compound (**9**) were recovered (yield = 81 %).

### Analysis:

¹H NMR (400 MHz, CDCl₃) δ 6.81 - 6.77 (m, 2H), 6.67 (s, 1H), 3.92 (m, 8H), 3.11 - 3.01 (m, 4H), 1.67 - 1.51 (m, 8H), 1.26 (m, 56H), 0.94 - 0.82 (t, *J* = 6.9 Hz, 12H).

³¹P NMR (162 MHz, CDCl₃) δ 26.40

HRMS: m/z theo: C₄₈H₉₂O₇P₂ 842.63 g.mol⁻¹, measured: C₄₈H₉₂O₇P₂Na: 866.235 g.mol⁻¹

### 2.3. Step 3 - Preparation of compound (10)

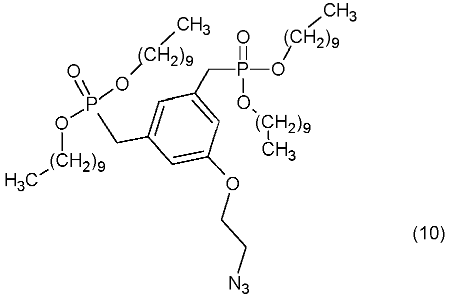

In a 100 mL flask were added 2.45 g (2.91 mmol - 1.0 Eq.) of compound **(9),** 0.25 g (4.36 mmol - 1.5 Eq.) of KOH and 49 g (0.3 mmol - 0.1 Eq.) of KI in 50 mL of toluene. The solution was stirred for 20 min and heated to 60°C. to dissolve the reagents. 1.05 g (4.36 mmol - 1.5 Eq.) of Linker 2 (as prepared above in step 1.7. of example 1) were added and the reaction was left to stir overnight.

The flask was cooled to room temperature, then the toluene was evaporated off under reduced pressure. The crude was then dissolved in DCM, and filtered through Celite. A chromatographic column was carried out DCM / MeOH (1/0 to 98/2 to 96/4). 2.1 g of compound (**10**) (79%) were thus recovered.

### Analysis:

¹H NMR (400 MHz, MeOD) δ 6.90 - 6.82 (m, 3H), 4.17 (t, *J* = 4.8 Hz, 2H), 3.98 (m, 8H), 3.63 - 3.56 (t, *J* = 4.9 Hz, 2H), 3.25 - 3.13 (d, *J* = 22 Hz, 4H), 1.62 (p, *J* = 6.6 Hz, 8H), 1.32 (m, 54H), 0.96 - 0.85 (t, *J* = 6.5 Hz, 12H).

¹³C NMR (101 MHz, MeOD) δ 160.00, 134.57, 134.45, 125.47, 115.96, 68.46, 67.77, 67.73, 67.70, 51.32, 34.31, 33.12, 32.94, 31.67, 31.64, 31.61, 30.75, 30.74, 30.51, 30.33, 26.71, 23.78, 14.48.

³¹P NMR (162 MHz, MeOD) δ 31.03.

### 2.4. Step 4 - Preparation of a dendritic molecule of formula (I-B)

To a solution of 237 mg (0.259 mmol - 1.1 Eq.) of compound (**10**) in 15 mL of EtOAc and 251 mg (0.1 Eq.) of Pd/C 10% as catalyst. The resulting mixture was purged with an atmosphere of hydrogen (five times), then vigorously stirred at RT for 5h. In a separate flask, 175 mg (0.236 mmol - 1.0 Eq.) of compound (**3**) as prepared above at step 1.3. of example 1 were dissolved in 15 mL of CH₂Cl₂ before 0.13 mL (0.71 mmol - 3.0 Eq.) of (COCl)₂ and 4 drops of DMF were added. The orange solution was stirred at RT for 5h. When TLC confirmed the full consumption of compound (**3**), the catalyst was filtered over Celite and the crude product was concentrated under reduced pressure at RT. The acyl chloride was simultaneously concentrated under reduced pressure, dissolved in CH₂Cl₂ then the crude primary amine and 0.11 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure to yield to an orange oil. Purification by flash chromatography (100% EtOAc then CH₂Cl₂/EtOAc/MeOH 80/12/8 to 6/0/4) were conducted to separate the pure and impure fractions, to afford the dendritic molecule of formula (**I-B**) (293 mg, 67%) as colorless oil.

### Analysis:

¹H NMR (400 MHz, MeOD) δ 7.23 (s, 2H), 6.85 (m, 2H), 4.26 - 4.13 (m, 6H), 4.08 - 3.90 (m, 8H), 3.88 - 3.85 (t, *J* = 4.2 Hz, 2H), 3.81 - 3.78 (m, 1H), 3.76 (t, *J* = 5.7 Hz, 1H), 3.71 (m, 4H), 3.67 - 3.57 (m, 19H), 3.55 - 3.49 (m, 4H), 3.37 - 3.31 (bs, 9H), 3.24 - 3.14 (ABₓ, *J* = 21.9 Hz, 4H), 1.67 - 1.55 (m, 8H), 1.29 (m, 55H), 0.90 (t, *J* = 6.9Hz, 12H).

³¹P NMR (162 MHz, MeOD) δ 27.14.

### EXAMPLE 3: Synthesis of a dendritic molecule of formula (I-C) according to the invention

In this example, a dendritic molecule of the following formula (I-C) was prepared:

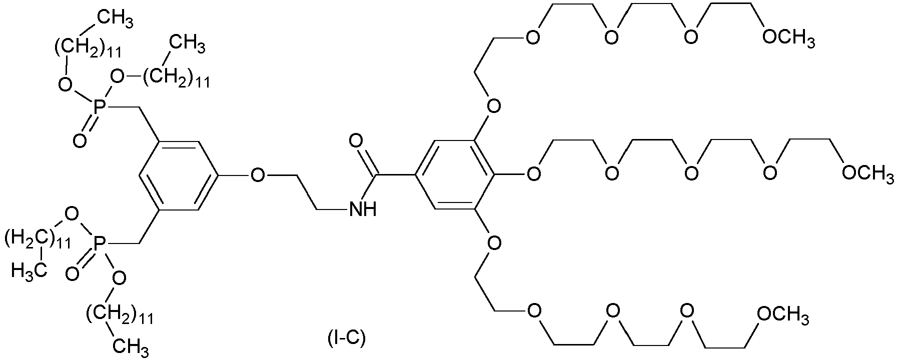

### 3.1. Step 1 - Preparation of compound (11)

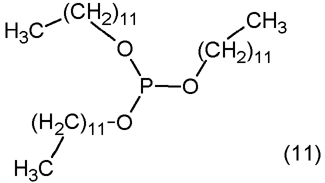

In a 250 mL two-necked flask connected to a Dean-Stark, 107.0 mL (469.0 mmol - 6.0 Eq.) of dodecanol was added, then the reaction setup was stirred and passed under a flow of argon for 10 min.

After that, the liquid was heated to 75°C and the flow replaced by 2 balloons of argon. 9.51 mL (78.2 mmol - 1.0 Eq.) of trimethylphosphite were added slowly from the syringe and then the setup was heated at 220°C for 6 h.

Then the setup was cooled to ambient temperature, and the Dean-Stark containing the methanol formed during the reaction removed. An orange oil was obtained.

Distillation under reduced pressure was used to separate the remaining dodecanol from the compound **(11)** thus formed, at 160°C.

43.0 g of compound **(11)** as a colourless oil were recovered (yield = 94%).

### Analysis:

¹H NMR (400 MHz, CDCl₃) δ 3.78 (m, 6H), 1.64 - 1.51 (m, 6H), 1.26 (m, 65H), 0.91 - 0.83 (t, *J* = 6.5 Hz, 9H).

¹³C NMR (101 MHz, CDCl₃) δ 62.97, 62.94, 62.44, 62.33, 32.88, 32.03, 31.29, 31.24, 29.79, 29.76, 29.73, 29.72, 29.58, 29.47, 29.44, 25.96, 25.89, 22.79, 14.17.

³¹P NMR (162 MHz, CDCl₃) δ 139.19.

### 3.2. Step 2 - Preparation of compound (12)

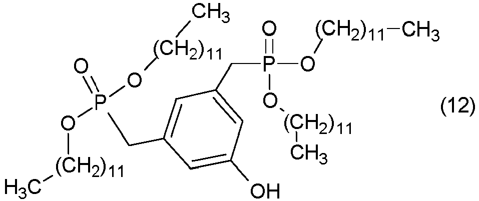

1.0 g (3.57 mmol - 1.0 Eq.) of compound **(4)** as prepared in step 1.4 of Example 1 above and 8.39 g (14.3 mmol - 4.0 Eq.) of compound **(11)** were added to a 100 mL flask. The reaction was stirred and heated to 140°C. overnight. A yellow-orange oil was obtained; a TLC was carried out (Petroleum ether / EtOAc 1/1).

The next day, after having cooled the solution, a Petroleum Ether / EtOAc chromatographic column was produced (1/1 to 2/3).

2.28 g of compound (**12**) were recovered (yield = 67 %).

### Analysis:

¹H NMR (400 MHz, CDCl₃) δ 6.80 (s, 2H), 6.66 (s, 1H), 3.92 (dq, *J* = 13.0, 8.0 Hz, 8H), 3.11 - 3.01 (m, 4H), 1.58 (t, *J* = 6.6 Hz, 8H), 1.25 (s, 75H), 0.88 (t, *J* = 6.9 Hz, 12H).

¹³C NMR (101 MHz, CDCl₃) δ 157.61, 132.68, 122.76, 115.79 (C, Ar), 66.45 (CH₂-O-R), 31.94, 30.61, 29.70, 29.68, 29.64, 29.59, 29.38, 29.25, 25.53, 23.84, 22.70 (CH₂), 14.12 (CH₃).

³¹P NMR (162 MHz, CDCl₃) δ 26.40.

C₄₈H₉₂NaO₇P₂ m/z calculated: 978,3898, m/Z found: 978,3990.

### 3.3. Step 3 - Preparation of compound (13)

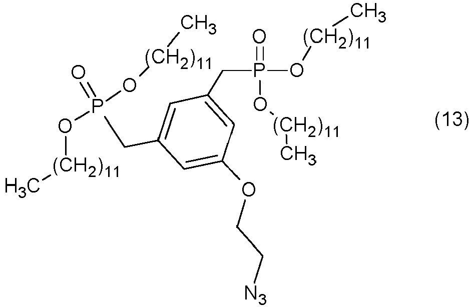

In a 100 mL flask were added 500 mg (0.523 mmol - 1.0 Eq.) of compound **(12),** 44.0 mg (0.785 mmol - 1.5 Eq.) of KOH and 26 mg (0.157 mmol - 0.1 Eq.) of KI in 25 mL of toluene. The solution was stirred for 20 min and heated to 60°C. to dissolve the reagents. 252.4 mg (0.785 mmol - 1.5 Eq.) of Linker 2 (as prepared above in step 1.7. of example 1) were added and the reaction was left to stir overnight.

The flask was cooled to room temperature, then the toluene was evaporated off under reduced pressure. The crude was then dissolved in DCM, and filtered through Celite. A chromatographic column was carried out DCM / MeOH (1/0 to 98/2 to 96/4). 500 mg of compound **(13)** (93%) were thus recovered.

### Analysis:

¹H NMR (400 MHz, CDCl₃) δ 6.87 - 6.68 (m, 3H), 4.13 (t, *J* = 5.0 Hz, 2H), 4.01 - 3.85 (m, 8H), 3.56 (d, *J* = 5.1 Hz, 2H), 3.08 (ABx, *J* = 21.9 Hz, 4H), 1.64 - 1.52 (m, 8H), 1.32 - 1.20 (m, 72H), 0.88 (t, *J* = 6.6 Hz, 12H).

³¹P NMR (162 MHz, CDCl₃) δ 25.94.

### 3.4. Step 4 - Preparation of a dendritic molecule of formula (I-C)

To a solution of 305 mg (0.297 mmol - 1.1 Eq.) of compound **(13)** in 15 mL of EtOAc were added 320 mg (0.1 Eq.) of Pd/C 10% as catalyst. The resulting mixture was purged with an atmosphere of hydrogen (five times), then vigorously stirred at RT for 5h. In a separate flask, 200 mg (0.270 mmol - 1.0 Eq.) of compound **(3)** as prepared above at step 1.3. of example 1 were dissolved in 15 mL of CH₂Cl₂ before 0.15 mL (0.81 mmol - 3.0 Eq.) of (COCl)₂ and 4 drops of DMF were added. The orange solution was stirred at RT for 5h. When TLC confirmed the full consumption of compound **(3),** the catalyst was filtered over Celite and the crude product was concentrated under reduced pressure at RT. The acyl chloride was simultaneously concentrated under reduced pressure, dissolved in CH₂Cl₂ then the crude primary amine and 0.11 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced Purification by flash chromatography (100% EtOAc then CH₂Cl₂/EtOAc/MeOH 80/12/8 to 6/0/4) were conducted to separate the pure and impure fractions, to afford the dendritic molecule of formula **(I-C)** (265 mg, 58%) as colorless oil.

### Analysis:

¹H NMR (400 MHz, CDCl₃) δ 7.12 (s, 2H), 6.74 (m, 3H), 4.19 (m, 4H), 4.09 (t, *J* = 5.8 Hz, 2H), 3.90 (m, 8H), 3.82 (t, *J* = 4.8 Hz, 2H), 3.78 - 3.71 (m, 4H), 3.61 (m, 19H), 3.51 - 3.48 (m, 4H), 3.35 - 3.27 (m, 9H), 3.10 - 2.97 (ABx, *J* = 21.8 Hz, 4H), 1.55 (h, *J* = 6.2 Hz, 8H), 1.22 (m, 76H), 0.84 (t, *J* = 6.4 Hz, 12H).

³¹P NMR (162 MHz, CDCl₃) δ 25.88.

### EXAMPLE 4: Synthesis of a dendritic molecule of formula (I-D) according to the invention

In this example, a dendritic molecule of the following formula **(I-D)** was prepared:

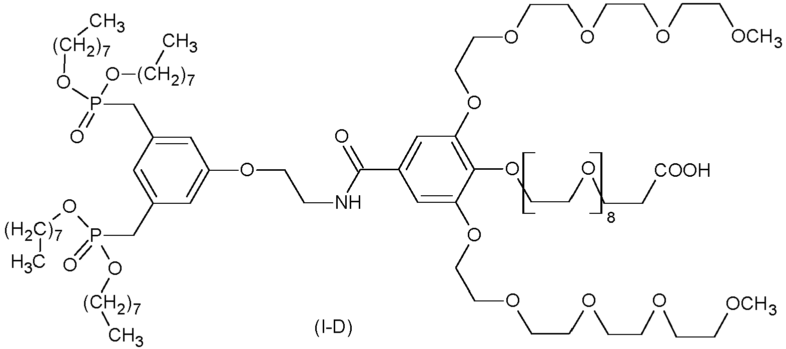

### 4.1. Step 1 - Preparation of compound (14)

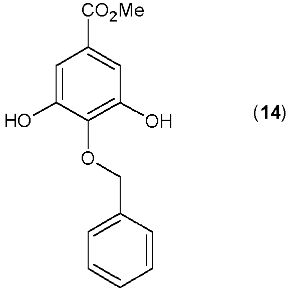

To a solution of 30 g (163 mmol - 1 Eq.) of methyl gallate in 150 mL of DMF were successively added 49.1 g (489 mmol - 4.5 Eq.) of KHCO₃, 0.136 g (0.82 mmol - 0.006 Eq.) of KI and 21.3 mL (163 mmol - 1.0 Eq.) of benzyl bromide (BnBr). The resulting mixture was stirred at RT for 36h.

The solids were filtered over Celite, the filtrate was acidified with an aqueous solution of HCl 2N (50.0 mL). The aqueous mixture was extracted with EtOAc (three times), the combined organic layer was washed with an aqueous saturated solution of NaHCO₃ (two times), brine (three times), dried over Na₂SO₄, filtered, concentrated under reduced pressure and dried under vacuum overnight. Purification with a chromatographic column (DCM/MeOH, 100:0 to 98:2 to 96:4 to 90:10) gave a translucent oil. The mixture was then dissolved with a small amount of AcOEt and Petroleum ether was poured slowly until precipitation occurred. Compound **(14)** was obtained as a white solid (16.5g, 38%)

### Analysis:

¹H NMR (400 MHz, MeOD) δ 7.53 - 7.44 (m, 2H, Ar-H), 7.30 (qd, *J* = 6.9, 3.7 Hz, 3H, Ar-H), 7.01 (d, *J* = 1.3 Hz, 2H, Ar-H), 5.14 (s, 2H, Ar-C*H*₂), 3.82 (d, *J* = 1.5 Hz, 3H, O-C*H*₃).

¹³C NMR (101 MHz, MeOD) δ 168.49, 151.90, 139.62, 138.80, 129.87, 129.19, 129.16, 129.11, 126.44 (12C, *C*-Ar), 110.07 (1C, C-*C*OOR), 75.12 (1C, Ar-*C*H₂), 52.46 (1C, O-*C*H₃).

### 4.2. Step 2 - Preparation of compound (15)

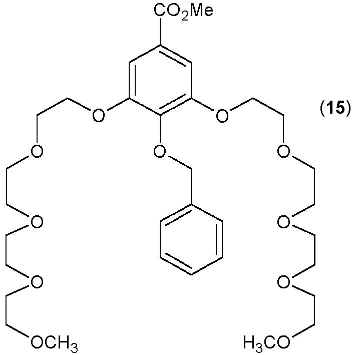

To a solution of 5.81 g (16.1 mmol - 2.2 Eq.) of compound **(14)** in 150.0 mL of acetone were added 3.22 g (23.4 mmol - 3.2 Eq.) of K₂CO₃,0.12 g (0.73 mmol - 0.4 Eq.) of KI and 2.0 g (7.3 mmol - 1.0 Eq.) of compound **(1)** as prepared in step 1.1 of example 1. The resulting solution was heated to reflux overnight.

The reaction mixture was cooled to RT, the solvent was removed, solids were suspended in CH₂Cl₂, filtered over Celite, washed with an aqueous solution of Na₂S₂O₃ 2N and brine, dried over Na₂SO₄, filtered and concentrated under reduced pressure. Purification by flash chromatography (CH₂Cl₂/MeOH 98/2 to 96/4 to 9/1) gave 3.3 g (71%) of compound **(15)** in the form of a yellowish oil.

### Analysis:

¹H NMR (400 MHz, Chloroform-d) δ 7.46 (d, 2H : Ar-*H*), 7.35 - 7.20 (m, 5H : Ar-*H*), 5.09 (s, 2H : -C*H*₂-OAr), 4.15 (dd, *J* = 5.6, 4.2 Hz, 4H: -O-C*H₂*-R), 3.86 (s, 3H : C*H*₃-O-C=O-R), 3.85 - 3.81 (m, 4H : -O-CH₂-C*H₂*-R), 3.70 - 3.66 (m, 4H : -O-C*H₂*-R), 3.63 - 3.56 (m, 16H : -O-C*H₂*-R), 3.51 - 3.47 (m, 4H : -O-C*H₂*-R), 3.33 (s, 6H : C*H₃*-O-).

¹³C NMR (101 MHz, CDCl₃) δ 169.26, 153.95, 142.97, 139.17, 129.74, 129.22, 129.01, 127.17, 109.67, 75.95, 72.93, 71.83, 71.65, 71.57, 71.50, 71.32, 70.81, 69.98, 59.06.

### 4.3. Step 3 - Preparation of compound (16)

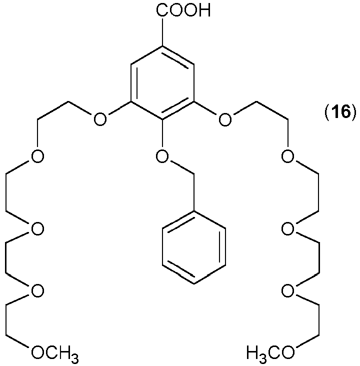

1.01 g (25.22 mmol - 5.0 Eq.) of NaOH were added to a solution of 3.3 g (5.04 mmol - 1.0 Eq.) of compound (**15**) in 27 mL of MeOH and 3 mL of distilled water. The orange solution was stirred at RT overnight.

The solvent was removed, the crude product was suspended in CH₂Cl₂, quenched with an aqueous solution of HCl 2N, the aqueous layer was extracted with CH₂Cl₂ (five times), the combined organic layer was dried over Na₂SO₄, filtered and concentrated under reduced pressure to yield to compound **(16)** (3.05 g, 95%) as a light yellow oil.

### Analysis:

¹H NMR (400 MHz, Chloroform-d) δ 7.46 (d, 2H : Ar-*H*), 7.35 - 7.20 (m, 5H : Ar-*H*), 5.09 (s, 2H : -C*H*₂-OAr), 4.15 (dd, *J* = 5.6, 4.2 Hz, 4H: -O-C*H₂*-R), 3.85 - 3.81 (m, 4H : -O-CH₂-C*H₂*-R), 3.70 - 3.66 (m, 4H : -O-C*H₂*-R), 3.63 - 3.56 (m, 16H : -O-C*H₂*-R), 3.51 - 3.47 (m, 4H : -O-C*H₂*-R), 3.33 (s, 6H : C*H₃*-O-).

### 4.4. Step 4 - Preparation of compound (17)

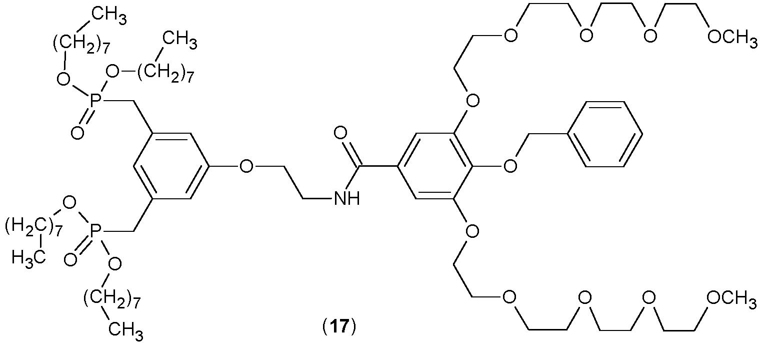

To a solution of 1.67 g (2.09 mmol - 1.0 Eq.) of compound (**7**) as prepared at step 1.8 of example 1 in 40 mL of EtOAc were added 1.1 g (0.1 Eq.) of Pd/C 10% as catalyst. The resulting mixture was purged with an atmosphere of hydrogen (five times), then vigorously stirred at RT for 5h. When TLC confirmed the full consumption of compound **(7),** the catalyst was filtered over Celite and the crude product was concentrated under reduced pressure at RT. In parallel, compound **(16)** (1.00 eq., 2.09 mmol, 1.34 g) was dissolved in DCM (67.0 mL), then 1-ethyl-3-(3-dimethylaminopropyl)carbodiimide (EDC) (1.50 eq., 3.13 mmol, 612 mg), and DMAP (0.10 eq., 0.21 mmol, 26 mg) were added, and the reaction stirred for 10 min before adding the crude primary amine. The resulting mixture was stirred at RT overnight. Monitoring by TLC DCM/MeOH, 6:4, KMnO4. The reaction mixture was concentrated under reduced pressure, the crude was purified by flash chromatography (SiO₂ 40.0 g, solid deposit in DCM, eluent: DCM/MeOH 100/0 to 90/10) and give expected product **(17)** (2.55 g, 89% yield) as a colorless oil

### Analysis:

1H NMR (400 MHz, MeOD) :δ 7.54 - 7.52 (m, 2H, Ar-*H*), 7.36 - 7.28 (m, 3H, Ar-*H*), 7.21 (s, 2H, Ar-*H*), 6.86 - 6.85 (m, 3H, Ar-*H*), 5.49 (s, 3H, ?), 5.10 (s, 2H, O-C*H*₂-Ar), 4.19 (t, 4H, Ar-O-C*H*₂-CH₂-O, *J*), 4.18 (t, 2H, O-C*H*₂-CH₂-N, *J*), 3.99 - 3.94 (m, 9H, P-O-C*H*₂-CH₂), 3.88 - 3.86 (m, 4H, Ar-O-CH₂-C*H*₂-O), 3.77 - 3.74 (t, 2H, O-CH₂-C*H*₂-N, J), 3.72 - 3.69 (m, 4H, O-CH₂-C*H*₂-O), 3.63 - 3.54 (m, 17H, O-CH₂-C*H*₂-O), 3.49 - 3.47 (m, 4H, ...), 3.31 (s, 6H, O-C*H*₃), 3.19 (d, 4H, P-C*H*₂, *J*), 1.62 - 1.58 (m, 9H, P-O-CH₂-C*H*₂-), 1.33 - 1.28 (m, 44H), 0.89 (t, 12H, CH₂-C*H*₃, *J*).

¹³C NMR (101 MHz, MeOD) δ 169.56, 160.38, 154.04, 141.70, 139.20, 130.67, 129.73, 129.22, 129.00, 115.98, 107.49, 75.94, 72.93, 71.83, 71.64, 71.56, 71.51, 71.32, 70.82, 69.99, 67.74, 67.71, 67.67, 67.54, 59.09, 54.80, 34.29, 32.98, 31.65, 31.62, 31.59, 30.69, 30.38, 30.26, 26.68, 23.73, 14.49.

³¹P NMR (400 MHz, Methano-d4): δ 27.16

### 4.5. Step 5 - Preparation of compound (18)

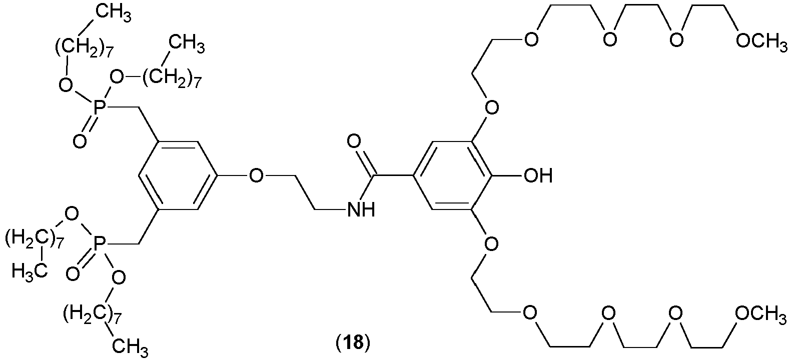

To a solution of 2.55g (1.83 mmol - 1.0 Eq.) of compound **(17)** in 40.0 mL of EtOAc were added 355.0 mg of Pd/C 10% as catalyst. The heterogeneous solution was purged with an atmosphere of hydrogen (five times), then vigorously stirred at RT overnight, the catalyst was filtered off over Celite, the crude product was concentrated under reduced pressure, to give 2.32compound **(18)** as a colorless oil (97%).

### Analysis:

¹H NMR (400 MHz, MeOD) δ 7.24 (s, 2H), 6.85 (m, 3H), 4.25 - 4.18 (m, 4H), 4.16 (t, *J* = 5.7 Hz, 2H), 4.13 - 4.05 (m, 1H), 4.06 - 3.90 (m, 8H), 3.91 - 3.84 (m, 4H), 3.78 - 3.69 (m, 6H), 3.68 - 3.58 (m, 19H), 3.55 - 3.47 (m, 4H), 3.33 (s, 6H), 3.24 - 3.13 (AB_{X}, *J* = 21.9 Hz, 4H), 1.59 (m, 8H), 1.37 - 1.20 (m, 44H), 0.90 (t, *J* = 6.85 Hz, 12H).

¹³C NMR (101 MHz, MeOD) δ 169.79, 148.23, 141.70, 125.33, 115.98, 108.36, 72.96, 71.66, 71.58, 71.53, 71.36, 70.77, 70.03, 67.74, 67.67, 61.53, 59.09, 58.32, 40.71, 34.28, 33.00, 31.66, 31.60, 30.67, 30.40, 30.27, 26.69, 23.74, 20.86, 18.37, 14.49, 14.46.

³¹P NMR (162 MHz, MeOD) δ 27.17.

### 4.6. Step 6 - Preparation of Linker 3 (L3)

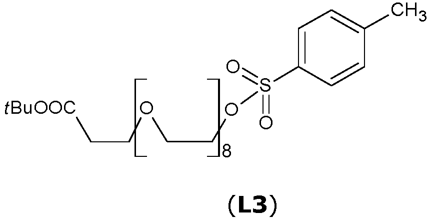

To 3.00 g (6.02 mmol - 1 Eq.) of t-Butyl 1-hydroxy-3,6,9,12,15,18,21,24-octaoxaheptacosan-27-oate in 50 mL of DCM, was added 1.69 mL (12.04 mmol - 2 Eq.) of Et₃N slowly, the mixture was stirred 10 min, then 2.32 g (12.04 mmol - 2 Eq.) de tosyl chloride was added slowly.

After the night TLC (EtP, AcOEt 7:3, KMnO₄) showed no traces of starting material. The mixture was concentrated, tosyl chloride salts were precipitated in AcOEt and filtered over celite pad, washed 3 times with AcOEt, to give the crude product (orange oil). It was then purified by Flash chromatography, Interchim-80g-50, liquid deposit in EtP/AcOEt (9:1), Eluent: 15 min EtP/AcOEt (9:1), 10 min EtP/AcOEt (8:2) with isocratic, and 10min EtP/AcOEt (7:3).

3.52 g of Linker 3 as a colourless oil was recovered (90%)

### Analysis:

¹H NMR (400 MHz, MeOD) δ 7.80 (d, *J* = 8.3 Hz, 2H), 7.45 (d, *J* = 8.2 Hz, 2H), 4.14 (t, *J* = 4.5 Hz, 2H), 3.77 - 3.50 (m, 32H), 2.51 - 2.43 (m, 5H), 1.45 (s, 9H).

### 4.7. Step 7 - Preparation of compound (19)

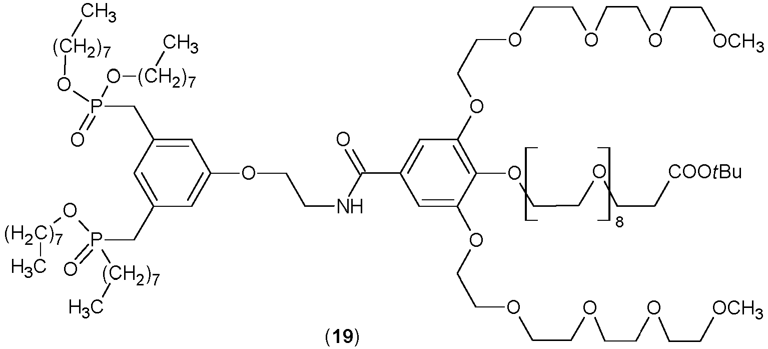

To a solution of 1.0 g (0.76 mmol - 1.0 Eq) of compound **(18)** in 25 mL of acetone, 0.48 g (0.84 mmol - 1.1 Eq.) of **Linker 3,** 51.3 mg (0.30 mmol - 0.4 Eq.) of KI and 340 mg (2.45 mmol - 3.2 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure. Purification by flash chromatography (CH₂Cl₂/MeOH 95/5 to 8/2 to 6/4) afforded 827 mg of pure compound (**19**) as a yellowish oil (65% yield).

### Analysis:

¹H NMR (400 MHz, MeOD) δ 7.26 (s, 2H), 6.86 (m, 3H), 4.29 - 4.22 (m, 6H), 4.18 (t, *J* = 5.6 Hz, 2H), 3.97 (m, 8H), 3.91 - 3.86 (m, 4H), 3.78 (m, 3H), 3.73 - 3.50 (m, 48H), 3.34 (s, 6H), 3.25 - 3.11 (AB_{X}, *J* = 21.9 Hz, 4H), 2.48 (t, *J* = 6.2 Hz, 2H), 1.67 - 1.55 (m, 8H), 1.41 - 1.23 (m, 44H), 0.94 - 0.86 (t, *J* = 6.7 Hz 12H).

¹³C NMR (101 MHz, MeOD) δ 172.76, 169.27, 153.62, 140.98, 134.50, 131.15, 116.01, 107.25, 81.74, 73.60, 72.94, 71.51, 71.44, 71.29, 71.25, 71.23, 71.15, 71.10, 71.05, 70.99, 70.51, 69.57, 67.85, 67.75, 67.68, 67.51, 59.16, 58.32, 40.80, 37.18, 34.27, 32.99, 31.67, 31.61, 30.40, 30.27, 28.41, 26.69, 23.74, 18.37, 14.49.

³¹P NMR (162 MHz, MeOD) δ 27.17.

### 4.8. Step 8 - Preparation of a dendritic molecule of formula (I-D)

In a flask containing 826 mg (0.48 mmol - 1.0 Eq.) dissolved in 8 mL of DCM, 1.23 mL (15.9 mmol, 33 Eq). of trifluoroacetic acid (TFA) was added dropwise. The mixture was stirred for 2h, then evaporated under reduced pressure, affording the dendritic molecule of formula **(I-D)** as a colorless oil (91%).

### Analysis:

¹H NMR (400 MHz, MeOD) δ 7.25 (s, 2H), 6.86 (m, 3H), 4.28 - 4.20 (m, 6H), 4.17 (t, *J* = 5.6 Hz, 2H), 3.97 (m, 8H), 3.92 - 3.85 (m, 4H), 3.82 - 3.73 (m, 4H), 3.76 - 3.53 (m, 45H), 3.55 - 3.48 (m, 4H), 3.33 (s, 6H), 3.20 (ABx, *J* = 21.9 Hz, 4H), 2.54 (t, *J* = 6.2 Hz, 2H), 1.67 - 1.53 (m, 8H), 1.31 (m, 44H), 0.90 (t, *J* = 7.0 Hz, 11H).

¹³C NMR (101 MHz, MeOD) δ 167.99, 152.31, 129.42, 114.57, 106.20, 72.22, 71.56, 70.20, 70.10, 70.00, 69.92, 69.88, 69.85, 69.26, 68.43, 66.38, 66.35, 66.28, 66.13, 57.73, 39.41, 34.37, 32.87, 31.59, 30.26, 30.20, 28.99, 28.87, 25.29, 22.33, 13.08.

³¹P NMR (162 MHz, MeOD) δ 27.17.

### EXAMPLE 5: Synthesis of a dendritic molecule of formula (I-E) according to the invention

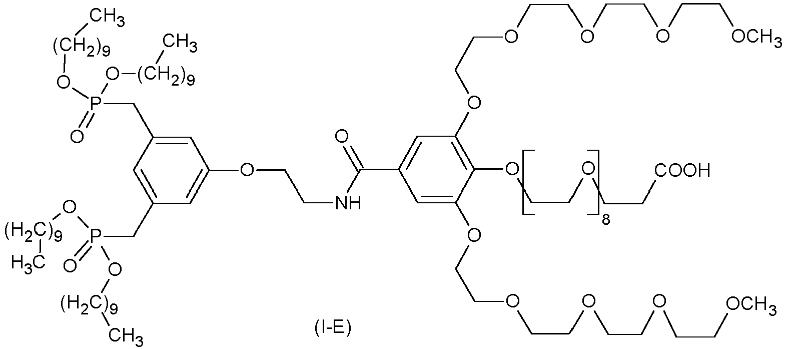

### 5.1. Step 1 - Preparation of compound (20)

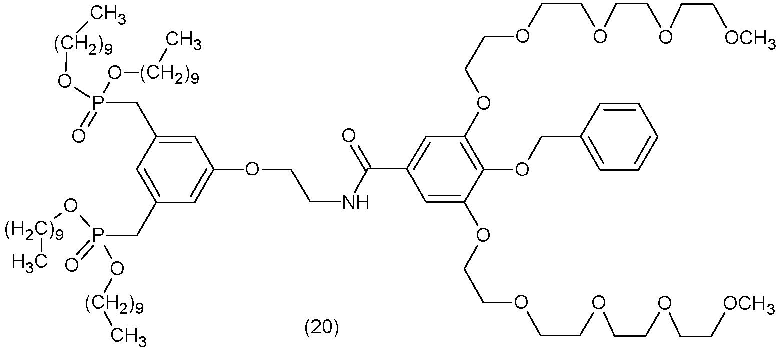

To a solution of 1.64 g (1.80 mmol - 1.0 Eq.) of compound **(10)** as prepared at step 3 of example 4 in 40 mL of EtOAc were added 960 mg (0.1 Eq.) of Pd/C 10% as catalyst. The resulting mixture was purged with an atmosphere of hydrogen (five times), then vigorously stirred at RT for 5 h.

When TLC confirmed the full consumption of compound **(10),** the catalyst was filtered over Celite and the crude product was concentrated under reduced pressure at RT. In parallel, compound **(16)** (1.1 eq, 1.98 mmol, 1.27 g) was dissolved in DCM (60.0 mL), then EDC (1.50 eq, 2.7 mmol, 529 mg), and DMAP (0.10 eq, 0.18 mmol, 22 mg) were added, and the reaction stirred for 10 min before adding the crude primary amine. The resulting mixture was stirred at RT overnight. Monitoring by TLC DCM/MeOH, 6:4, KMnO4. The reaction mixture was concentrated under reduced pressure, the crude was purified by flash chromatography

(SiO₂ 40.0 g, solid deposit in DCM, eluent: DCM/MeOH 100/0 to 90/10) and give expected product (**20**) (2.41 g, 88% yield) as a colorless oil

### Analysis:

¹H NMR (400 MHz, MeOD) δ 7.46 - 7.39 (s, 2H), 7.28 - 7.14 (m, 3H), 7.11 (s, 2H), 6.79 - 6.72 (m, 3H), 5.00 (s, 2H), 4.13 - 4.03 (m, 6H), 3.91 - 3.81 (m, 8H), 3.79 - 3.75 (m, 4H), 3.65 (t, *J* = 5.6 Hz, 2H), 3.62 - 3.59 (m, 4H), 3.55 - 3.42 (m, 17H), 3.40 - 3.36 (m, 4H), 3.21 (s, 6H), 3.09 (AB_{X}, J = 21.4 Hz, 4H), 1.56 - 1.44 (m, 8H), 1.31 - 1.11 (m, 56H), 0.79 (t, *J* = 6.7 Hz, 12H).

¹³C NMR (101 MHz, MeOD) δ 169.55, 160.40, 154.06, 141.75, 139.23, 130.67, 129.73, 129.22, 128.99, 116.01, 107.50, 75.94, 72.95, 71.85, 71.67, 71.58, 71.53, 71.35, 70.84, 70.00, 67.75, 67.68, 67.55, 59.09, 54.80, 40.78, 34.31, 33.11, 32.94, 31.67, 31.61, 30.75, 30.73, 30.67, 30.52, 30.32, 26.70, 23.78, 14.50.

³¹P NMR (162 MHz, MeOD) δ 27.15.

### 5.2. Step 2 - Preparation of compound (21)

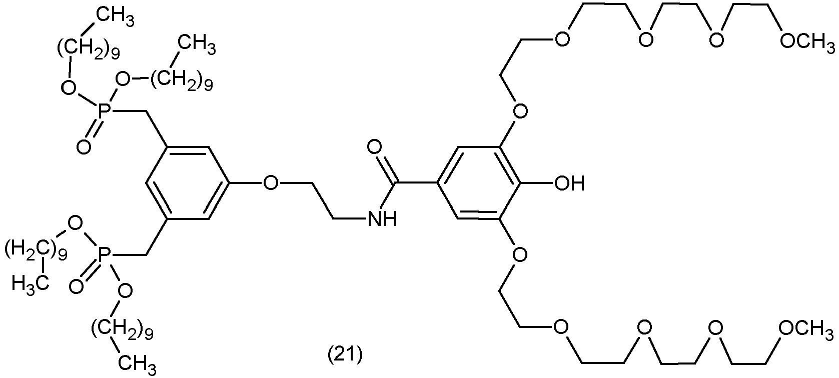

To a solution of 2.41 g (1.6 mmol - 1.0 Eq.) of compound (**20**) in 40 mL of EtOAc were added 340.0 mg of Pd/C 10% as catalyst. The heterogeneous solution was purged with an atmosphere of hydrogen (five times), then vigorously stirred at RT for 5h, the catalyst was filtered off over Celite, the crude product was concentrated under reduced pressure, to give 2.25 g of compound **(21)** as a colorless oil (99%).

### Analysis:

¹H NMR (400 MHz, MeOD) δ 7.24 (s, 2H), 6.85 (m, 3H), 4.22 - 4.20 (m, 4H), 4.16 (t, *J* = 5.7 Hz, 2H), 4.13 - 4.07 (m, 2H), 4.01 - 3.92 (m, 8H), 3.89 - 3.85 (m, 3H), 3.77 - 3.71 (m, 6H), 3.69 - 3.57 (m, 26H), 3.54 - 3.47 (m, 4H), 3.33 (s, 6H), 3.19 (AB_{X}, *J* = 22.0 Hz, 4H), 1.66 - 1.55 (m, 8H), 1.29 (m, 56H), 0.90 (d, *J* = 7.3 Hz, 12H).

¹³C NMR (101 MHz, MeOD): δ 172.97, 169.77, 148.23, 141.71, 125.32, 115.99, 108.37, 72.97, 71.67, 71.58, 71.54, 71.36, 70.77, 70.03, 67.75, 67.67, 61.53, 59.10, 58.32, 40.71, 34.30, 33.12, 32.94, 31.67, 31.64, 31.61, 30.75, 30.73, 30.67, 30.52, 30.32, 26.70, 23.78, 20.86, 18.37, 14.50, 14.47.

³¹P NMR (162 MHz, MeOD) δ 27.16.

### 5.3. Step 3 - Preparation of compound (22)

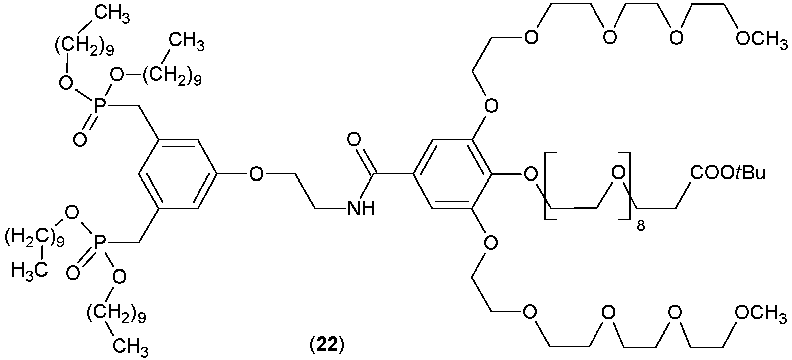

To a solution of 1.01 g (0.705 mmol - 1.0 Eq.) of compound (**21**) in 25 mL of acetone, 0.440 g (0.775 mmol - 1.1 Eq.) of Linker 3 as prepared at step 6 of example 48 mg (0.282 mmol - 0.4 Eq.) of KI and 313 mg (2.26 mmol - 3.2 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure. Purification by flash chromatography (CH₂Cl₂/MeOH 95/5 to 8/2 to 6/4) afforded 736 mg of compound (**22**) as a yellowish oil (59% yield).

### Analysis:

¹H NMR (400 MHz, MeOD) δ 7.27 (s, 2H), 6.86 (m, 3H), 4.30 - 4.22 (m, 6H), 4.18 (t, *J* = 5.6 Hz, 2H), 4.03 - 3.91 (m, 8H), 3.93 - 3.86 (m, 4H), 3.81 - 3.75 (m, 3H), 3.76 - 3.46 (m, 48H), 3.34 (s, 6H), 3.20 (AB_{X}, *J* = 21.9 Hz, 4H), 2.48 (t, *J* = 6.2 Hz, 2H), 1.60 (m, 8H), 1.45 (s, 9H), 1.29 (s, 56H), 0.90 (t, *J* = 6.7 Hz, 12H).

¹³C NMR (101 MHz, MeOD) δ 153.63, 116.02, 107.32, 81.74, 72.95, 71.52, 71.46, 71.30, 70.53, 69.60, 67.86, 67.75, 59.15, 58.32, 37.19, 33.11, 31.67, 31.61, 30.75, 30.73, 30.51, 30.32, 28.40, 26.70, 23.77, 14.49.

³¹P NMR (162 MHz, MeOD) δ 27.16.

### 5.4. Step 4 - Preparation of a dendritic molecule of formula (I-E)

In a flask containing 736 mg (0.406 mmol - 1.0 Eq.) of compound (**22**) dissolved in DCM, 30 Eq. of TFA was added dropwise. The mixture was stirred for 2h, then evaporated under reduced pressure, affording 645 mg of the dendritic molecule of formula **(I-E)** as a colorless oil (93%)

### Analysis:

¹H NMR (400 MHz, MeOD) δ 7.15 (s, 2H), 6.76 (m, 3H), 4.18 - 4.11 (m, 6H), 4.07 (t, *J* = 5.6 Hz, 2H), 3.94 - 3.82 (m, 8H), 3.81 - 3.77 (m, 4H), 3.71 - 3.45 (m, 48H), 3.44 - 3.40 (m, 4H), 3.23 (s, 6H), 3.10 (AB_{X}, *J* = 21.8 Hz, 4H), 2.48 (t, *J* = 6.2 Hz, 2H), 1.50 (q, *J* = 6.7 Hz, 8H), 1.30 - 1.14 (m, 56H), 0.84 - 0.73 (m, 12H).

### EXAMPLE 6: Synthesis of a dendritic molecule of formula (I-F) according to the invention

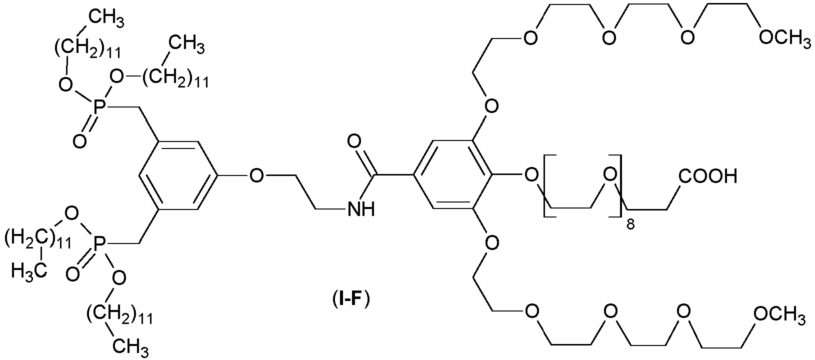

### 6.1. Step 1 - Preparation of compound (23)

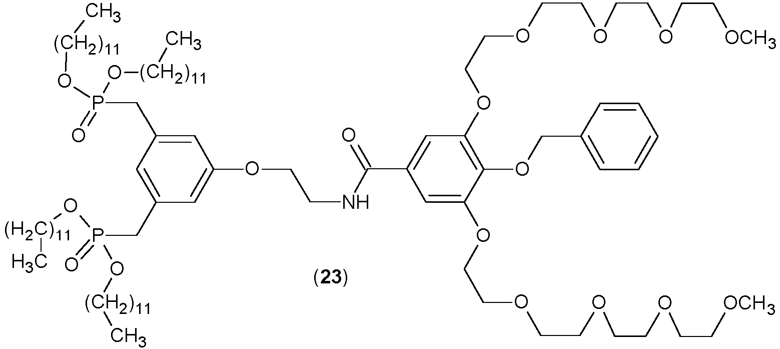

To a solution of 300 mg (0.312 mmol - 1.1 Eq.) of compound **(13),** as prepared at step 3 of example 3, in 15 mL of EtOAc were added 120 mg (0.1 Eq.) of Pd/C 10% as catalyst. The resulting mixture was purged with an atmosphere of hydrogen (five times), then vigorously stirred at RT for 5h. In a separate flask, 200 mg (0.260 mmol - 1.0 Eq.) of compound **(16),** as prepared in step 3 of example 4, were dissolved in 10 mL of CH₂Cl₂ before 0.1 mL (1.3 mmol - 3.0 Eq.) of (COCl)₂ and 4 drops of DMF were added. The orange solution was stirred at RT for 5h. When TLC confirmed the full consumption of compound **(13),** the catalyst was filtered over Celite and the crude product was concentrated under reduced pressure at RT. The acyl chloride was simultaneously concentrated under reduced pressure, dissolved in CH₂Cl₂ then the crude primary amine and 0.16 mL (0.916 mmol - 2.3 Eq.) of DIPEA were successively added at 0°C. The resulting solution was stirred at RT overnight.

The reaction mixture was diluted with brine. The aqueous layer was extracted with CH₂Cl₂ (five times), the combined organic layer was dried over Na₂SO₄, filtered and concentrated under reduced pressure. Purification by flash chromatography (100% EtOAc then CH₂Cl₂/EtOAc/MeOH 80/12/8 to 6/0/4) were conducted to separate the pure and impure fractions, to afford compound **(23)** (487.2 mg, 81%) as colorless oil.

### Analysis:

¹H NMR (400 MHz, MeOD) δ 7.24 (s, 2H), 6.85 (m, 3H), 4.22 - 4.20 (m, 4H), 4.16 (t, *J* = 5.7 Hz, 2H), 4.13 - 4.07 (m, 2H), 4.01 - 3.92 (m, 8H), 3.89 - 3.85 (m, 3H), 3.77 - 3.71 (m, 6H), 3.69 - 3.57 (m, 26H), 3.54 - 3.47 (m, 4H), 3.33 (s, 6H), 3.19 (ABₓ, *J* = 22.0 Hz, 4H), 1.66 - 1.55 (m, 8H), 1.29 (m, 78H), 0.90 (d, *J* = 7.3 Hz, 12H).

¹³C NMR (101 MHz, MeOD) δ 173.10, 169.67, 148.33, 141.71, 125.32, 116.02, 108.47, 72.97, 71.67, 71.58, 71.54, 71.36, 70.83, 70.03, 67.75, 67.67, 61.53, 59.10, 58.32, 40.71, 34.30, 33.12, 32.94, 31.67, 31.64, 31.61, 30.75, 30.73, 30.67, 30.52, 30.32, 26.70, 23.78, 20.86, 18.37, 14.50, 14.47.

³¹P NMR (162 MHz, MeOD) δ 27.16.

### 6.2. Step 2 - Preparation of compound (24)

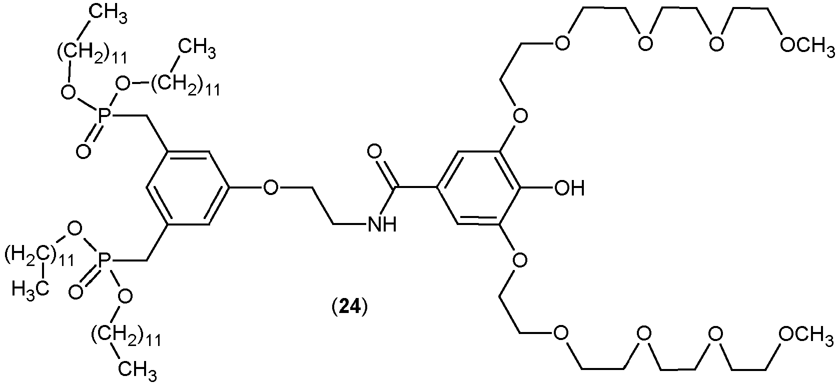

To a solution of 382 mg (0.236 mmol - 1.0 Eq.) of compound **(23)** in 40 mL of EtOAc were added 150.0 mg of Pd/C 10% as catalyst. The heterogeneous solution was purged with an atmosphere of hydrogen (five times), then vigorously stirred at RT for 5h, the catalyst was filtered off over Celite, the crude product was concentrated under reduced pressure, to give 320 mg of compound **(24)** as a colorless oil (89%).

### Analysis:

¹H NMR (400 MHz, MeOD) δ 7.24 (s, 2H), 6.85 (m, 3H), 4.25 - 4.18 (m, 4H), 4.16 (t, *J* = 5.7 Hz, 2H), 4.13 - 4.05 (m, 1H), 4.06 - 3.90 (m, 8H), 3.91 - 3.84 (m, 4H), 3.78 - 3.69 (m, 6H), 3.68 - 3.58 (m, 19H), 3.55 - 3.47 (m, 4H), 3.33 (s, 6H), 3.24 - 3.13 (ABₓ, J = 21.9 Hz, 4H), 1.59 (m, 8H), 1.37 - 1.20 (m, 78H), 0.90 (t, J = 6.85 Hz, 12H).

¹³C NMR (101 MHz, MeOD) δ 169.82, 148.27, 141.74, 125.30, 116.0, 108.40, 72.93, 71.71, 71.60, 71.53, 71.33, 70.77, 70.03, 67.74, 67.67, 61.53, 59.09, 58.35, 40.73, 34.27, 33.02, 31.66, 31.60, 30.67, 30.40, 30.27, 26.69, 23.74, 20.86, 18.39, 14.51, 14.46.

³¹P NMR (162 MHz, MeOD) δ 27.17.

### 6.3. Step 3 - Preparation of compound (25)

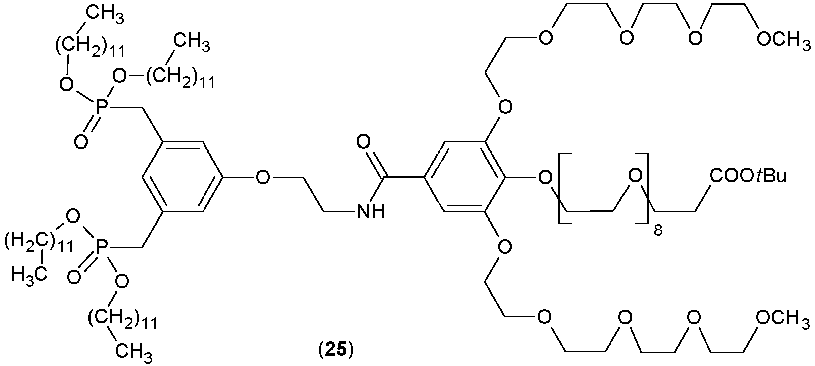

To a solution of 320 mg (0.209 mmol - 1.0 Eq.) of compound **(24)** in 40 mL of acetone, 0.145 g (0.219 mmol - 1.05 Eq.) of Linker 3 as prepared at step 6 of example 4, 33 mg (0.02 mmol - 0.1 Eq.) of KI and 45 mg (0.315 mmol - 1.5 Eq.) of K₂CO₃ were successively added. The resulting reaction mixture was heated to reflux overnight.

The reaction mixture was cooled to RT, the solvent was removed, the crude product was suspended in CH₂Cl₂, the solids were filtered off; the crude product was concentrated under reduced pressure to yield an orange oil. Purification by flash chromatography (CH₂Cl₂/MeOH 95/5 to 8/2 to 6/4) afforded 321 mg of compound **(25)** as a yellowish oil (76% yield).

### Analysis:

¹H NMR (400 MHz, MeOD) δ 7.27 (s, 2H), 6.86 (m, 3H), 4.30 - 4.22 (m, 6H), 4.18 (t, *J* = 5.6 Hz, 2H), 4.03 - 3.91 (m, 8H), 3.93 - 3.86 (m, 4H), 3.81 - 3.75 (m, 3H), 3.76 - 3.46 (m, 48H), 3.34 (s, 6H), 3.20 (ABₓ, *J* = 21.9 Hz, 4H), 2.48 (t, *J* = 6.2 Hz, 2H), 1.60 (m, 8H), 1.45 (s, 8H), 1.29 (s, 78H), 0.90 (t, *J* = 6.7 Hz, 12H).

¹³C NMR (101 MHz, MeOD) δ 153.63, 116.02, 107.32, 81.74, 72.95, 71.52, 71.46, 71.30, 70.53, 69.60, 67.86, 67.75, 59.15, 58.32, 37.19, 33.11, 31.67, 31.61, 30.75, 30.73, 30.51, 30.32, 28.40, 26.70, 23.77, 14.49.

³¹P NMR (162 MHz, MeOD) δ 27.16.

## Claims

1. A dendritic molecule of formula (I) below: 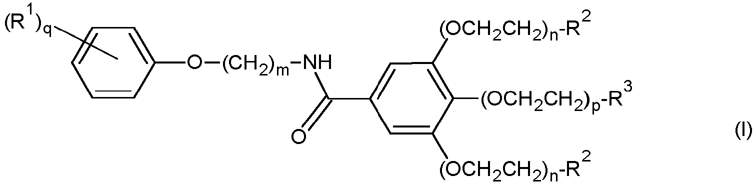 wherein :
- R¹ is a phosphonate group of the following formula (PG): 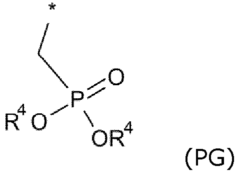 in which each of R⁴ represents a linear alkyl radical having at least 4 carbon atoms and the star represents the attachment point of said group of formula (PG) to the phenyl cycle;
- each of R² represents a linear alkyloxy radical having from 1 to 20 carbon atoms;
- R³ represents a linear alkyloxy radical having from 1 to 20 carbon atoms, a carboxyl group or a group -COOtBu in which tBu means *ter*-butyl;
- n is an integer ranging from 1 to 16;
- p is an integer ranging from 1 to 16;
- m is an integer ranging from 1 to 4, preferably m = 1 or 2 and more preferably m = 2; and
- q = 2.

2. The dendritic molecule according to claim 1, wherein q = 2 and R¹ represents a phosphonate group of formula (PG) in which each of R⁴ represents an alkyl group having from 4 to 12 carbon atoms.

3. The dendritic molecule according to claim 2, wherein each of R⁴ represents an alkyl group selected among octyl, decanyl, and dodecanyl.

4. The dendritic molecule according to anyone of claims 1 to 3, wherein n is an integer ranging from 4 to 6 inclusively.

5. The dendritic molecule according to any one of claims 1 to 4, wherein p is an integer ranging from 4 to 10 inclusively.

6. The dendritic molecule according to any one of claims 1 to 5, wherein each of R² represents a methyloxy group.

7. The dendritic molecule according to any one of claims 1 to 6, wherein R³ represents a methyloxy group, a carboxyl group or a group -COO*t*Bu in which tBu means ter-butyl.

8. The dendritic molecule according to any one of claims 1 to 7, wherein said dendritic molecule is selected among compounds of formulae (I-A) to (I-F) whose significations of R¹ to R⁴, m, n, p and q are given in the following Table 1:
**TABLE 1**
| **Cpds** | **R¹** | **R²** | **R³** | **R⁴** | **n** | **p** | **m** | **q** |
|---|---|---|---|---|---|---|---|---|
| (I-A) | PG | -OCH₃ | -OCH₃ | -(CH₂)₇-CH₃ | 4 | 4 | 2 | 2 |
| (I-B) | PG | -OCH₃ | -OCH₃ | -(CH₂)₉-CH₃ | 4 | 4 | 2 | 2 |
| (I-C) | PG | -OCH₃ | -OCH₃ | -(CH₂)₁₁-CH₃ | 4 | 4 | 2 | 2 |
| (I-D) | PG | -OCH₃ | -COOH | -(CH₂)₇-CH₃ | 4 | 9 | 2 | 2 |
| (I-E) | PG | -OCH₃ | -COOH | -(CH₂)₉-CH₃ | 4 | 9 | 2 | 2 |
| (I-F) | PG | -OCH₃ | -COOH | -(CH₂)₁₁-CH₃ | 4 | 9 | 2 | 2 |

9. A process for the preparation of dendritic molecules of formula (I) as defined in claim 1 in which R¹ is a phosphonate group of formula (PG), q = 2, n = p and R² and R³ are identical and represent a linear alkyloxy radical having from 1 to 20 carbon atoms, said process comprising at least one step of reacting a compound of formula (II) below: 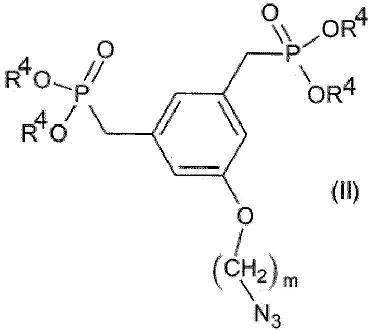
wherein R⁴ and m have the same signification as in formula (I), with a compound of formula (III) below: 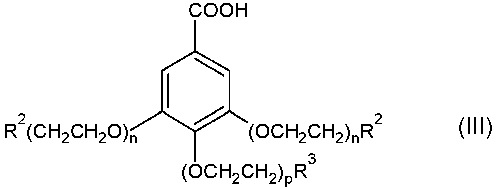
wherein n = p and have the same definition as in formula (I) and further wherein R² and R³ are identical and represent an alkyloxy group as defined in formula (I), to lead to the corresponding compound of formula (I).

10. The process according to claim 9 wherein compounds of formula (III) are prepared according to a process comprising at least the following steps:
- reacting an alcohol of formula R⁵-(OCH₂CH₂)ₙOH (IV) in which R⁵ represents a linear alkyloxy radical having from 1 to 20 carbon atoms with tosyl chloride to obtain a compound of formula (VI) below: wherein 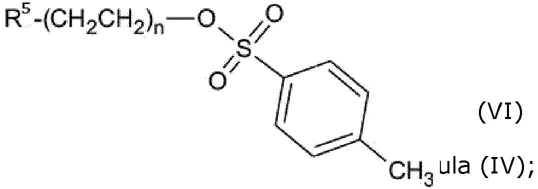
- reacting said compound of formula (VI) with methyl gallate to obtain a compound of formula (VIII) below: 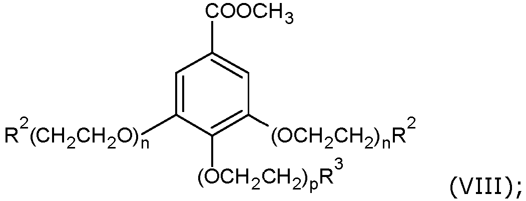 wherein n, p, R² and R³ have the same meaning as in formula (III); and
- unprotecting the carboxyl function of compound of formula (VIII) thus obtained to lead to the corresponding compound of formula (III).

11. Process according to claim 9, wherein compounds of formula (II) are prepared according to a process comprising at least the following steps:
- reacting an alcohol of formula R⁴-OH (IX) wherein R⁴ has the same meaning as in formula (I) with trimethylphosphite to obtain a compound of formula (XI) below: 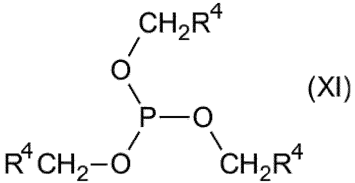 wherein R⁴ has the same meaning as in formula (I);
- reacting the compound of formula XI thus obtained with 3,5-bis(bromomethyl)phenol to obtain a compound of formula (XIII) below: 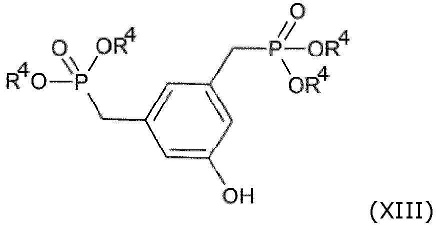 wherein R⁴ has the same meaning as in formula (I); and
- reacting the compound of formula (XIII) thus obtained with a compound of formula (XIV) below: 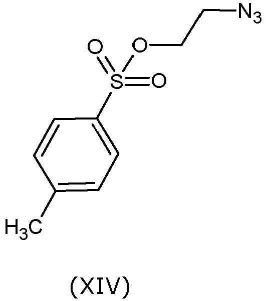 in which m has the same meaning as in formula (I) to obtain the corresponding compound of formula (II).

12. A process for the preparation of dendritic molecules of formula (I) as defined in claim 1 and in which R¹ is a phosphonate group (PG), q = 2, n and p are identical or different, R² is an alkyloxy group as defined in formula (I) and R³ is a carboxyl group or a group -COOtBu, said process comprising at least the following steps:
- reacting methyl gallate with benzyl bromide to obtain a compound of formula (XV) below: 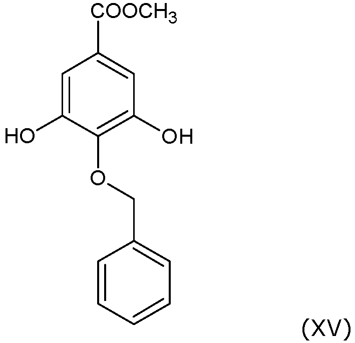
- independently reacting an alcohol of formula R²-(CH₂CH₂)ₙOH (IV') wherein R² and n have the same meaning as in formula (I) as defined in claim 1 with tosyl chloride to obtain a compound of formula (VI') below: 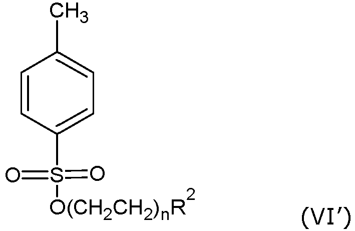 wherein R² and n have the same meaning as in formula (I) as defined in claim 1;
- reacting the compound of formula (XV) and the compound of formula (VI') thus obtained to obtain a compound of formula (XVI) below: 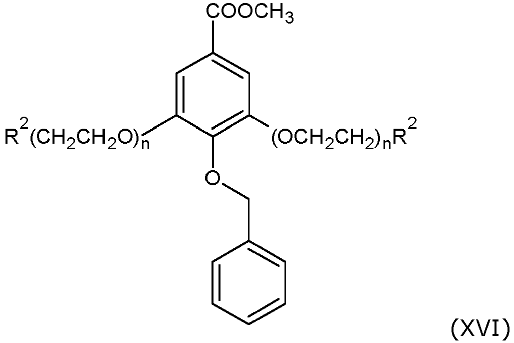 wherein R² and n have the same meaning as in formula (I) as defined in claim 1;
- unprotecting the carboxylic function of the compound of formula (XVI) thus obtained to lead to the compound of formula (XVII) below: 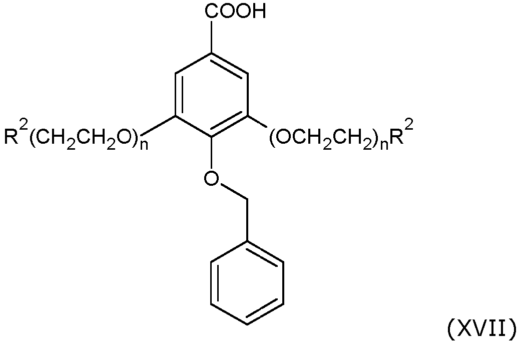 wherein R² and n have the same meaning as in formula (I) as defined in claim 1;
- reacting the compound of formula (XVII) thus obtained with a compound of formula (II) as defined in claim 10 to obtain a compound of formula (XVIII) below: 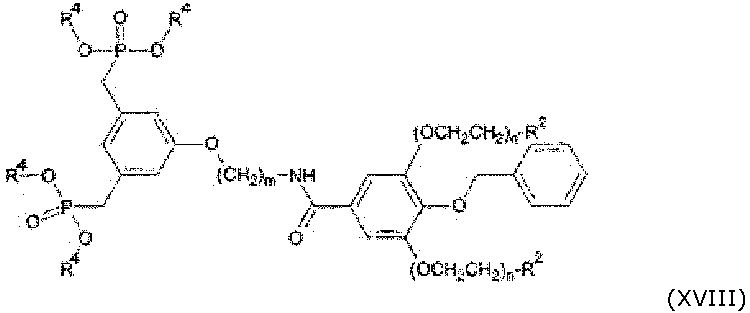 wherein R⁴, R², m and n have the same meaning as in formula (I) as defined in claim 1;
- hydrolyzing the benzyl group of the compound of formula (XVIII) thus obtained to obtain a compound of formula (XIX) below: 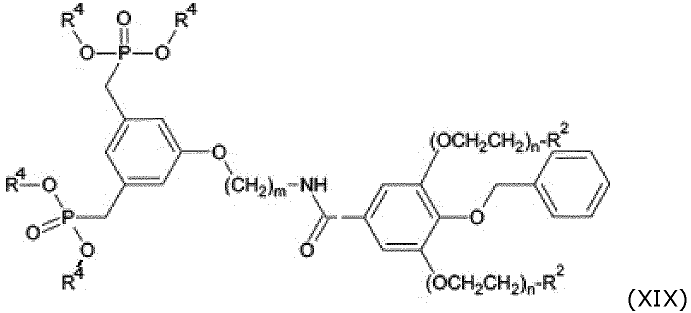 wherein R⁴, R², m and n have the same meaning as in formula (I) as defined in claim 1;
- reacting the compound of formula (XIX) thus obtained with a compound of formula (XX) below: 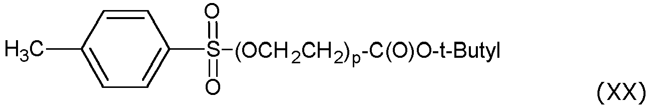
wherein p has the same meaning as in formula (I) as defined in claim 1 and has a value which is identical or different to the value of n in the compound of formula (XIX) as defined above, to obtain a compound of formula (XXI) below: 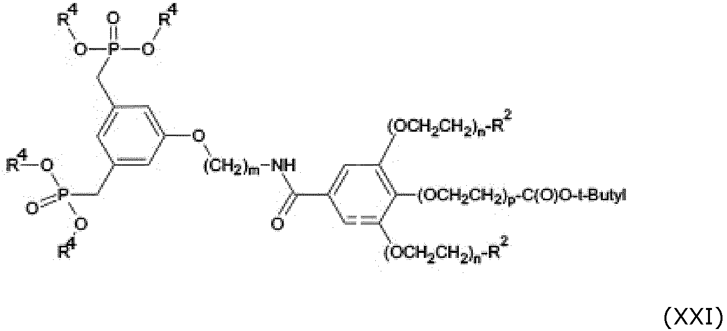
wherein R⁴, R², m, n and p have the same meaning as in formula (I) above and p has the same meaning as in formula (I) and has a value which is identical or different to the value of n in the compound of formula (XIX) as defined above and which corresponds to a particular compound of formula (I) in which R³ is a group -COOtBu ; and optionally;
- unprotecting the carboxylic function of compound of formula (XXI) thus obtained to lead to the corresponding dendritic molecule of formula (I) in which R³ is a group -COOH.

13. The process according to claim 12, wherein compounds of formula (XX) are prepared according to a process comprising the step of reacting tosyl chloride with a compound of formula (XXII):
OHCH₂CH₂-(OCH₂CH₂)ₚ₋₁-C(O)O-*t*-Butyl (XXII)
wherein p has the same meaning as in formula (I) as defined in claim 1.

14. Use of a dendritic molecule of formula (I) as defined in any one of claims 1 to 8, as a drug carrier.

15. A dendritic molecule of formula (I) as defined in any one of claims 1 to 8, in combination with at least one imaging agent, for its use as contrast agent for medical imaging or diagnosis platforms.

## Patentansprüche

1. Dendritisches Molekül der nachstehenden Formel (I): 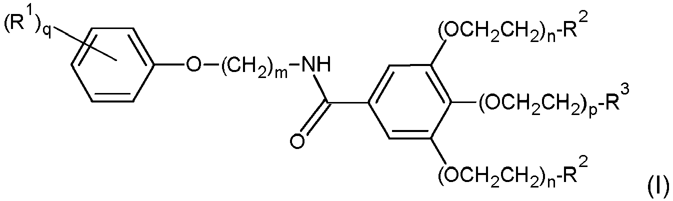 wobei:
- R¹ eine Phosphonatgruppe der folgenden Formel (PG) ist: 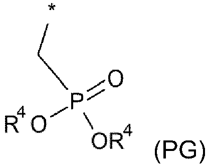 wobei jedes R⁴ ein lineares Alkylradikal mit mindestens 4 Kohlenstoffatomen darstellt und der Stern den Bindungspunkt der besagten Gruppe der Formel (PG) an dem Phenylring darstellt;
- jedes R² ein lineares Alkyloxyradikal mit 1 bis 20 Kohlenstoffatomen darstellt;
- R³ ein lineares Alkyloxyradikal mit 1 bis 20 Kohlenstoffatomen, einer Carboxylgruppe oder einer Gruppe -COOtBu darstellt, wobei tBu ter-Butyl bedeutet;
- n eine ganze Zahl im Bereich von 1 bis 16 ist;
- p eine ganze Zahl im Bereich von 1 bis 16 ist;
- m eine ganze Zahl im Bereich von 1 bis 4 ist, vorzugsweise m = 1 oder 2 und mehr bevorzugt m = 2; und
- q = 2.

2. Dendritisches Molekül nach Anspruch 1, wobei q = 2 ist und R¹ eine Phosphonatgruppe der Formel (PG) darstellt, wobei jedes R⁴ eine Alkylgruppe mit 4 bis 12 Kohlenstoffatomen darstellt.

3. Dendritisches Molekül nach Anspruch 2, wobei jedes R⁴ eine Alkylgruppe darstellt, ausgewählt aus Octyl, Decanyl und Dodecanyl.

4. Dendritisches Molekül nach einem der Ansprüche 1 bis 3, wobei n eine ganze Zahl im Bereich von 4 bis einschließlich 6 ist.

5. Dendritisches Molekül nach einem der Ansprüche 1 bis 4, wobei p eine ganze Zahl im Bereich von 4 bis einschließlich 10 ist.

6. Dendritisches Molekül nach einem der Ansprüche 1 bis 5, wobei jedes R² eine Methyloxygruppe darstellt.

7. Dendritisches Molekül nach einem der Ansprüche 1 bis 6, wobei R³ eine Methyloxygruppe, eine Carboxylgruppe oder eine Gruppe -COOtBu darstellt, wobei tBu ter-Butyl bedeutet.

8. Dendritisches Molekül nach einem der Ansprüche 1 bis 7, wobei besagtes dendritisches Molekül ausgewählt ist aus Verbindungen der Formeln (I-A) bis (I-F), deren Bedeutungen von R¹ bis R⁴, m, n, p und q in der folgenden Tabelle 1 angegeben sind:
**TABELLE 1**
| **Verbind gn.** | **R¹** | **R²** | **R³** | **R⁴** | **n** | **p** | **m** | **q** |
|---|---|---|---|---|---|---|---|---|
| (I-A) | PG | -OCH₃ | -OCH₃ | -(CH₂)₇-CH₃ | 4 | 4 | 2 | 2 |
| (I-B) | PG | -OCH₃ | -OCH₃ | -(CH₂)₉-CH₃ | 4 | 4 | 2 | 2 |
| (I-C) | PG | -OCH₃ | -OCH₃ | -(CH₂)₁₁-CH₃ | 4 | 4 | 2 | 2 |
| (I-D) | PG | -OCH₃ | -COOH | -(CH₂)₇-CH₃ | 4 | 9 | 2 | 2 |
| (I-E) | PG | -OCH₃ | -COOH | -(CH₂)₉-CH₃ | 4 | 9 | 2 | 2 |
| (I-F) | PG | -OCH₃ | -COOH | -(CH₂)₁₁-CH₃ | 4 | 9 | 2 | 2 |

9. Prozess zur Herstellung von dendritischen Molekülen der Formel (I), wie in Anspruch 1 definiert, wobei R¹ eine Phosphonatgruppe der Formel (PG), q = 2, n = p ist und R² und R³ identisch sind und ein lineares Alkyloxyradikal mit 1 bis 20 Kohlenstoffatomen darstellen, wobei besagter Prozess mindestens einen Schritt der Reaktion einer Verbindung der folgenden Formel (II) umfasst: 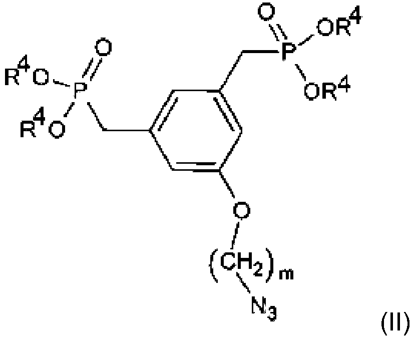
wobei R⁴ und m die gleiche Bedeutung wie in Formel (I) haben, mit einer Verbindung der nachstehenden Formel (III): 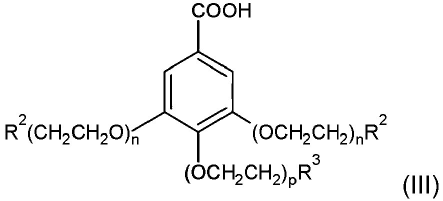
wobei n = p ist und die gleiche Definition wie in Formel (I) hat und wobei ferner R² und R³ identisch sind und eine Alkyloxygruppe wie in Formel (I) definiert darstellen, um zur entsprechenden Verbindung der Formel (I) zu führen.

10. Prozess nach Anspruch 9, wobei Verbindungen der Formel (III) nach einem Prozess hergestellt werden, der mindestens die folgenden Schritte umfasst:
- Reagieren eines Alkohols der Formel R⁵-(OCH₂CH₂)ₙOH (IV), wobei R⁵ ein lineares Alkyloxyradikal mit 1 bis 20 Kohlenstoffatomen darstellt, mit Tosylchlorid, um eine Verbindung der nachstehenden Formel (VI) zu erhalten: 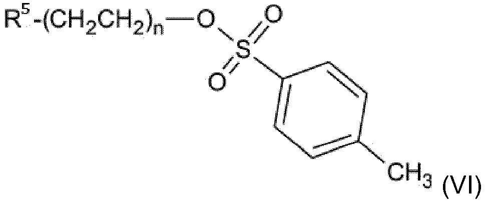
- Reagieren besagter Verbindung der Formel (VI) mit Methylgallat, um eine Verbindung der nachstehenden Formel (VIII) zu erhalten: 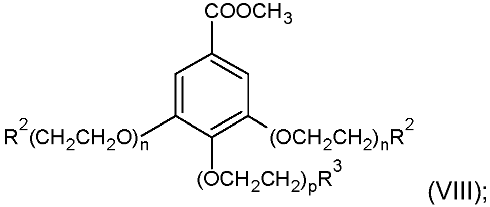 wobei n, p, R² und R³ dieselbe Bedeutung wie in Formel (III) haben; und
- Entschützen der Carboxylfunktion der so erhaltenen Verbindung der Formel (VIII), um zu der entsprechenden Verbindung der Formel (III) zu führen.

11. Prozess nach Anspruch 9, wobei Verbindungen der Formel (II) nach einem Prozess hergestellt werden, der mindestens die folgenden Schritte umfasst:
- Reagieren eines Alkohols der Formel R⁴-OH (IX), wobei R⁴ dieselbe Bedeutung wie in Formel (I) hat, mit Trimethylphosphit, um eine Verbindung der nachstehenden Formel (XI) zu erhalten: 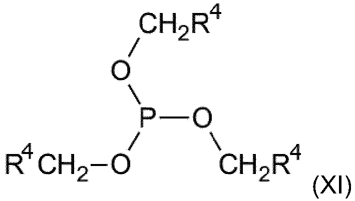 wobei R⁴ dieselbe Bedeutung wie in Formel (I) hat;
- Reagieren der so erhaltenen Verbindung der Formel XI mit 3,5-Bis(brommethyl)phenol, um eine Verbindung der nachstehenden Formel (XIII) zu erhalten: 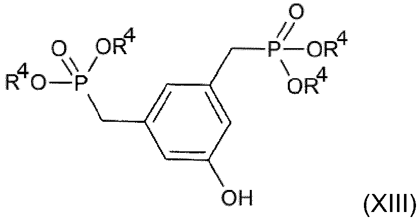 wobei R⁴ dieselbe Bedeutung wie in Formel (I) hat; und
- Reagieren der so erhaltenen Verbindung der Formel (XIII) mit einer Verbindung der nachstehenden Formel (XIV): 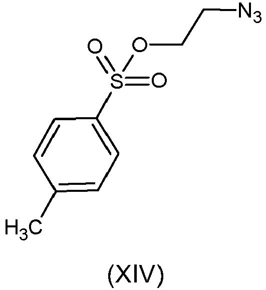 wobei m dieselbe Bedeutung wie in Formel (I) hat, um die entsprechende Verbindung der Formel (II) zu erhalten.

12. Prozess zur Herstellung dendritischer Moleküle der Formel (I), wie in Anspruch 1 definiert, wobei R¹ eine Phosphonatgruppe (PG) ist, q = 2, n und p identisch oder unterschiedlich sind, R² eine Alkyloxygruppe ist, wie in Formel (I) definiert, und R³ eine Carboxylgruppe oder eine Gruppe -COOtBu ist, wobei besagter Prozess mindestens die folgenden Schritte umfasst:
- Reagieren von Methylgallat mit Benzylbromid, um eine Verbindung der nachstehenden Formel (XV) zu erhalten: 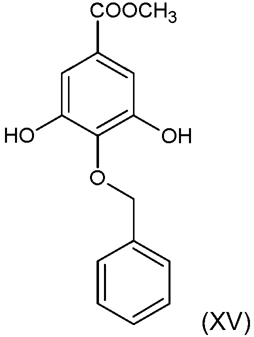
- unabhängiges Reagieren eines Alkohols der Formel R²-(CH₂CH₂)ₙOH (IV'), wobei R² und n dieselbe Bedeutung wie in Formel (I) haben, wie in Anspruch 1 definiert, mit Tosylchlorid, um eine Verbindung der nachstehenden Formel (VI') zu erhalten: 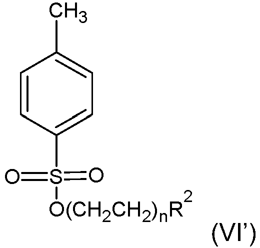 wobei R² und n dieselbe Bedeutung wie in Formel (I) haben, wie in Anspruch 1 definiert;
- Reagieren der Verbindung der Formel (XV) und der so erhaltenen Verbindung der Formel (VI'), um eine Verbindung der nachstehenden Formel (XVI) zu erhalten: 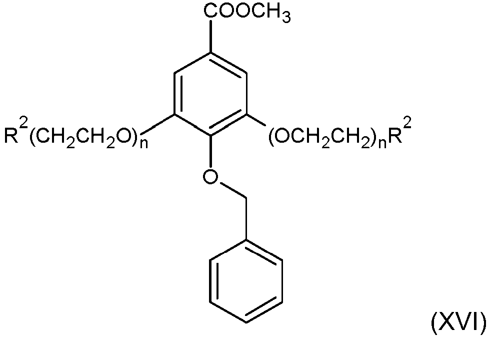 wobei R² und n dieselbe Bedeutung wie in Formel (I) haben, wie in Anspruch 1 definiert;
- Entschützen der Carboxylfunktion der so erhaltenen Verbindung der Formel (XVI), um zu der Verbindung der nachstehenden Formel (XVII) zu führen: 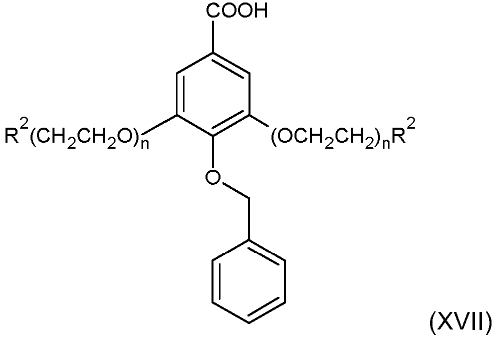 wobei R² und n dieselbe Bedeutung wie in Formel (I) haben, wie in Anspruch 1 definiert;
- Reagieren der so erhaltenen Verbindung der Formel (XVII) mit einer Verbindung der Formel (II), wie in Anspruch 10 definiert, um eine Verbindung der nachstehenden Formel (XVIII) zu erhalten: 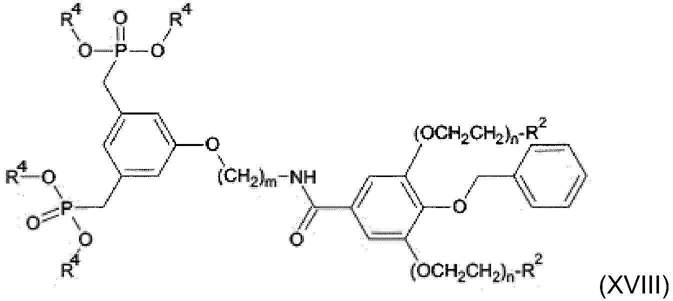 wobei R⁴, R², m und n dieselbe Bedeutung wie in Formel (I) haben, wie in Anspruch 1 definiert;
- Hydrolysieren der Benzylgruppe der so erhaltenen Verbindung der Formel (XVIII), um eine Verbindung der nachstehenden Formel (XIX) zu erhalten: 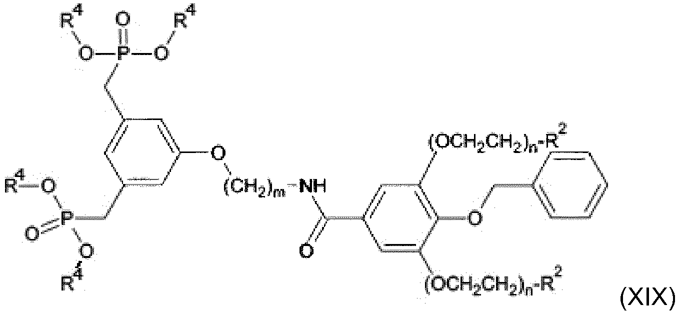 wobei R⁴, R², m und n dieselbe Bedeutung wie in Formel (I) haben, wie in Anspruch 1 definiert;
- Reagieren der so erhaltenen Verbindung der Formel (XIX) mit einer Verbindung der nachstehenden Formel (XX): 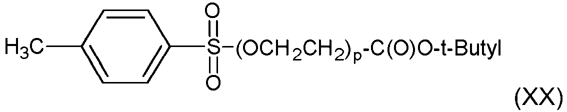
wobei p dieselbe Bedeutung wie in Formel (I) hat, wie in Anspruch 1 definiert, und einen Wert hat, der mit dem Wert von n in der oben definierten Verbindung der Formel (XIX) identisch oder unterschiedlich zu diesem ist, um eine Verbindung der nachstehenden Formel (XXI) zu erhalten: 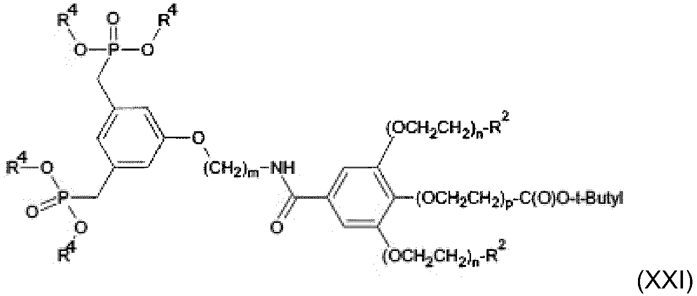
wobei R⁴, R², m, n und p dieselbe Bedeutung wie in der obigen Formel (I) haben und p dieselbe Bedeutung wie in Formel (I) hat und einen Wert hat, der mit dem Wert von n in der oben definierten Verbindung der Formel (XIX) identisch oder unterschiedlich zu diesem ist und der einer besonderen Verbindung der Formel (I) entspricht, in der R³ eine Gruppe -COOtBu ist; und optional;
- Entschützen der Carboxylfunktion der so erhaltenen Verbindung der Formel (XXI), um zu dem entsprechenden dendritischen Molekül der Formel (I) zu führen, in dem R³ eine Gruppe -COOH ist.

13. Prozess nach Anspruch 12, wobei Verbindungen der Formel (XX) nach einem Prozess hergestellt werden, der den Schritt des Reagierens von Tosylchlorid mit einer Verbindung der Formel (XXII) umfasst:
OHCH₂CH₂-(OCH₂CH₂)ₚ₋₁-C(O)O-*t*-Butyl (XXII)
wobei p dieselbe Bedeutung wie in Formel (I) hat, wie in Anspruch 1 definiert.

14. Verwendung eines dendritischen Moleküls der Formel (I), wie in einem der Ansprüche 1 bis 8 definiert, als Wirkstoffträger.

15. Dendritisches Molekül der Formel (I), wie in einem der Ansprüche 1 bis 8 definiert, in Kombination mit mindestens einem Bildgebungsmittel, zu seiner Verwendung als Kontrastmittel für medizinische Bildgebungs- oder Diagnoseplattformen.

## Revendications

1. Molécule dendritique de formule (I) ci-dessous : 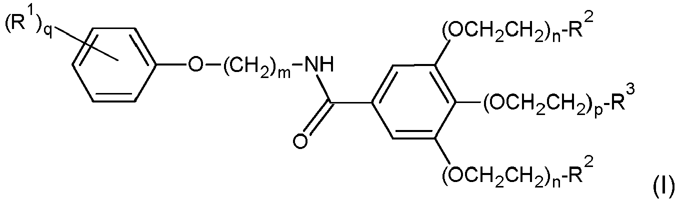 dans laquelle :
- R¹ est un groupe phosphonate de la formule suivante (PG) : 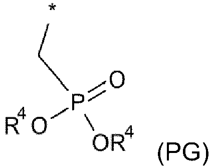 dans laquelle chacun de R⁴ représente un radical alkyle linéaire ayant au moins 4 atomes de carbone et l'astérisque représente le point d'attache dudit groupe de formule (PG) au cycle phényle ;
- chacun de R² représente un radical alkyloxy linéaire ayant de 1 à 20 atomes de carbone ;
- R³ représente un radical alkyloxy linéaire ayant de 1 à 20 atomes de carbone, un groupe carboxyle ou un groupe -COOtBu dans lequel tBu signifie ter-butyle ;
- n est un entier allant de 1 à 16 ;
- p est un entier allant de 1 à 16 ;
- m est un entier allant de 1 à 4, de préférence m = 1 ou 2 et plus préférablement m = 2 ; et
- q = 2.

2. Molécule dendritique selon la revendication 1, dans laquelle q = 2 et R¹ représente un groupe phosphonate de formule (PG) dans lequel chacun de R⁴ représente un groupe alkyle ayant de 4 à 12 atomes de carbone.

3. Molécule dendritique selon la revendication 2, dans laquelle chacun de R⁴ représente un groupe alkyle choisi parmi l'octyle, le décanyle et le dodécanyle.

4. Molécule dendritique selon l'une quelconque des revendications 1 à 3, dans laquelle n est un entier allant de 4 à 6 inclus.

5. Molécule dendritique selon l'une quelconque des revendications 1 à 4, dans laquelle p est un entier allant de 4 à 10 inclus.

6. Molécule dendritique selon l'une quelconque des revendications 1 à 5, dans laquelle chacun de R² représente un groupe méthyloxy.

7. Molécule dendritique selon l'une quelconque des revendications 1 à 6, dans laquelle R³ représente un groupe méthyloxy, un groupe carboxyle ou un groupe - COOtBu dans lequel tBu signifie ter-butyle.

8. Molécule dendritique selon l'une quelconque des revendications 1 à 7, dans laquelle ladite molécule dendritique est choisie parmi les composés de formules (I-A) à (I-F) dont les significations de R¹ à R⁴, m, n, p et q sont données dans le Tableau 1 suivant :
**TABLEAU 1**
| **Compo sés** | **R¹** | **R²** | **R³** | **R⁴** | **n** | **p** | **m** | **q** |
|---|---|---|---|---|---|---|---|---|
| (I-A) | PG | -OCH₃ | -OCH₃ | -(CH₂)₇-CH₃ | 4 | 4 | 2 | 2 |
| (I-B) | PG | -OCH₃ | -OCH₃ | -(CH₂)₉-CH₃ | 4 | 4 | 2 | 2 |
| (I-C) | PG | -OCH₃ | -OCH₃ | -(CH₂)₁₁-CH₃ | 4 | 4 | 2 | 2 |
| (I-D) | PG | -OCH₃ | -COOH | -(CH₂)₇-CH₃ | 4 | 9 | 2 | 2 |
| (I-E) | PG | -OCH₃ | -COOH | -(CH₂)₉-CH₃ | 4 | 9 | 2 | 2 |
| (I-F) | PG | -OCH₃ | -COOH | -(CH₂)₁₁-CH₃ | 4 | 9 | 2 | 2 |

9. Procédé de préparation de molécules dendritiques de formule (I) tel que défini dans la revendication 1, dans lequel R¹ est un groupe phosphonate de formule (PG), q = 2, n = p et R² et R³ sont identiques et représentent un radical alkyloxy linéaire ayant de 1 à 20 atomes de carbone, ledit procédé comprenant au moins une étape de réaction d'un composé de formule (II) ci-dessous : 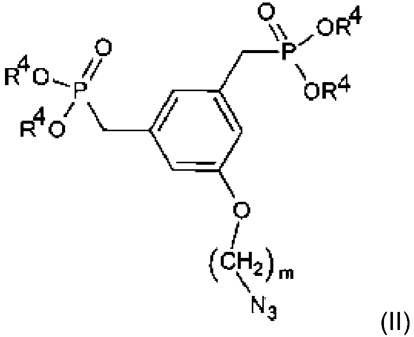
dans laquelle R⁴ et m ont la même signification que dans la formule (I), avec un composé de formule (III) ci-dessous : 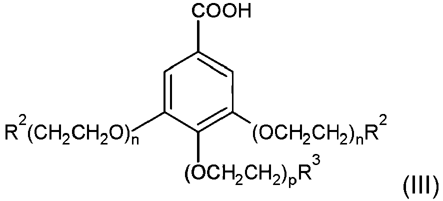
dans laquelle n = p et ont la même définition que dans la formule (I) et en outre dans lequel R² et R³ sont identiques et représentent un groupe alkyloxy tel que défini dans la formule (I), pour donner le composé correspondant de formule (I).

10. Procédé selon la revendication 9, dans lequel les composés de formule (III) sont préparés selon un procédé comprenant au moins les étapes suivantes :
- la mise en réaction d'un alcool de formule R⁵-(OCH₂CH₂)ₙOH (IV) dans laquelle R⁵ représente un radical alkyloxy linéaire ayant de 1 à 20 atomes de carbone avec du chlorure de tosyle pour obtenir un composé de formule (VI) ci-dessous : 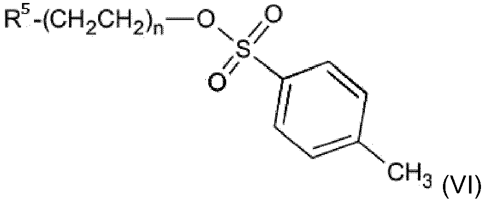
- la mise en réaction dudit composé de formule (VI) avec du gallate de méthyle pour obtenir un composé de formule (VIII) ci-dessous : 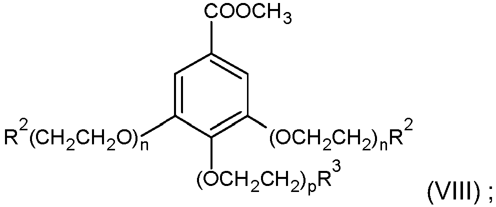 dans laquelle n, p, R² et R³ ont la même signification que dans la formule (III) ; et
- la déprotection de la fonction carboxyle du composé de formule (VIII) ainsi obtenu pour donner le composé correspondant de formule (III).

11. Procédé selon la revendication 9, dans lequel les composés de formule (II) sont préparés selon un procédé comprenant au moins les étapes suivantes :
- la mise en réaction d'un alcool de formule R⁴-OH (IX) dans laquelle R⁴ a la même signification que dans la formule (I) avec du triméthylphosphite pour obtenir un composé de formule (XI) ci-dessous : 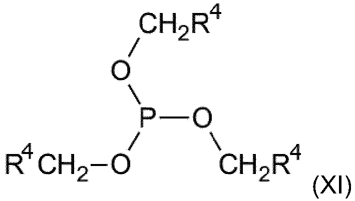 dans laquelle R⁴ a la même signification que dans la formule (I) ;
- la mise en réaction dudit composé de formule XI ainsi obtenu avec du 3,5-bis(bromométhyl)phénol pour obtenir un composé de formule (XIII) ci-dessous : 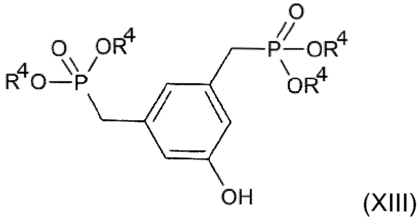 dans laquelle R⁴ a la même signification que dans la formule (I) ; et
- la mise en réaction du composé de formule (XIII) ainsi obtenu avec un composé de formule (XIV) ci-dessous : 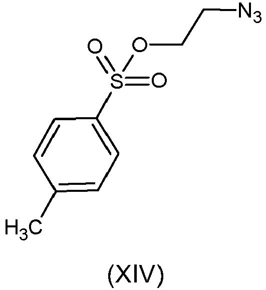 dans laquelle m a la même signification que dans la formule (I) pour obtenir le composé correspondant de formule (II).

12. Procédé de préparation de molécules dendritiques de formule (I) telle que définie dans la revendication 1 et dans lequel R¹ est un groupe phosphonate (PG), q = 2, n et p sont identiques ou différents, R² est un groupe alkyloxy tel que défini dans la formule (I) et R³ est un groupe carboxyle ou un groupe -COOtBu, ledit procédé comprenant au moins les étapes suivantes :
- la mise en réaction du gallate de méthyle avec le bromure de benzyle pour obtenir un composé de formule (XV) ci-dessous : 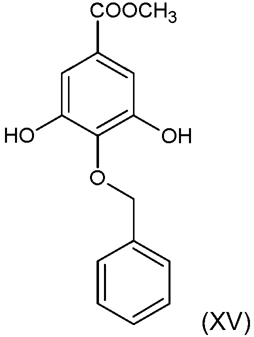
- la mise en réaction indépendamment d'un alcool de formule R²-(CH₂CH₂)ₙOH (IV') dans laquelle R² et n ont la même signification que dans la formule (I) telle que définie dans la revendication 1 avec du chlorure de tosyle pour obtenir un composé de formule (VI') ci-dessous : 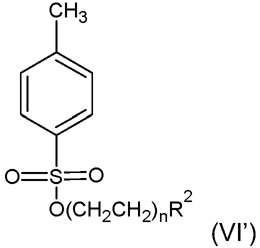 dans laquelle R² et n ont la même signification que dans la formule (I) telle que définie dans la revendication 1 ;
- la mise en réaction du composé de formule (XV) et du composé de formule (VI') ainsi obtenu pour obtenir un composé de formule (XVI) ci-dessous : 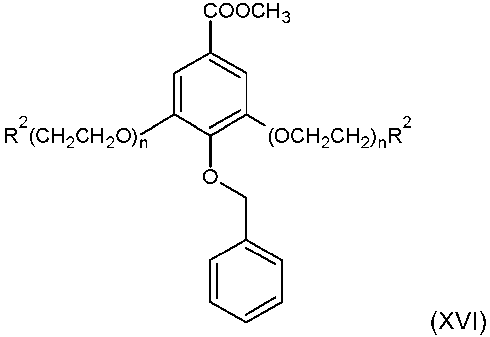 dans laquelle R² et n ont la même signification que dans la formule (I) telle que définie dans la revendication 1 ;
- la déprotection de la fonction carboxylique du composé de formule (XVI) ainsi obtenu pour donner le composé de formule (XVII) ci-dessous : 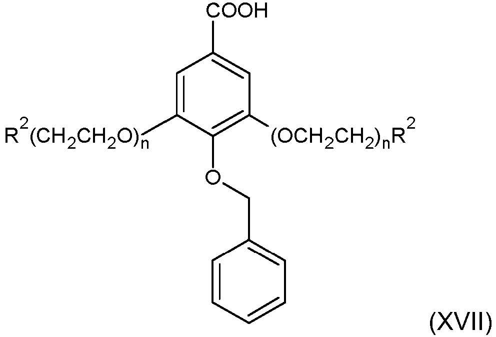 dans laquelle R² et n ont la même signification que dans la formule (I) telle que définie dans la revendication 1 ;
- la mise en réaction du composé de formule (XVII) ainsi obtenu avec un composé de formule (II) tel que défini dans la revendication 10 pour obtenir un composé de formule (XVIII) ci-dessous : 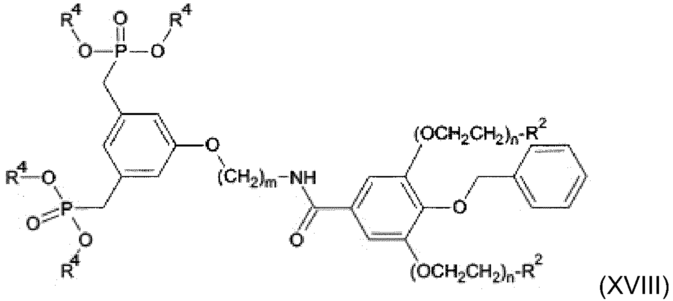 dans laquelle R⁴, R², m et n ont la même signification que dans la formule (I) telle que définie dans la revendication 1 ;
- l'hydrolysation du groupe benzyle du composé de formule (XVIII) ainsi obtenu pour obtenir un composé de formule (XIX) ci-dessous : 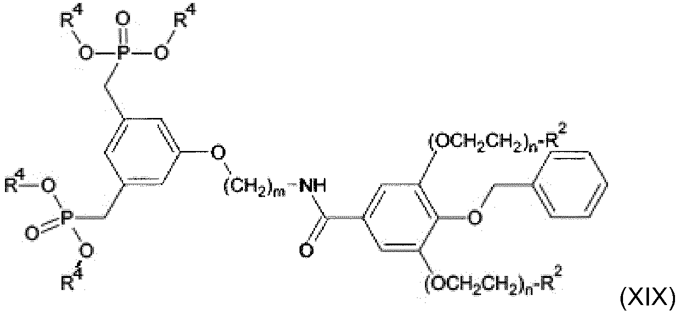 dans laquelle R⁴, R², m et n ont la même signification que dans la formule (I) telle que définie dans la revendication 1 ;
- la mise en réaction du composé de formule (XIX) ainsi obtenu avec un composé de formule (XX) ci-dessous : 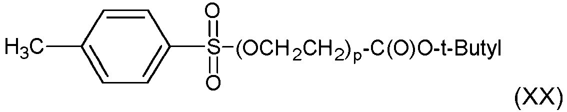
dans laquelle p a la même signification que dans la formule (I) telle que définie dans la revendication 1 et a une valeur identique ou différente de la valeur de n dans le composé de formule (XIX) tel que défini ci-dessus, pour obtenir un composé de formule (XXI) ci-dessous : 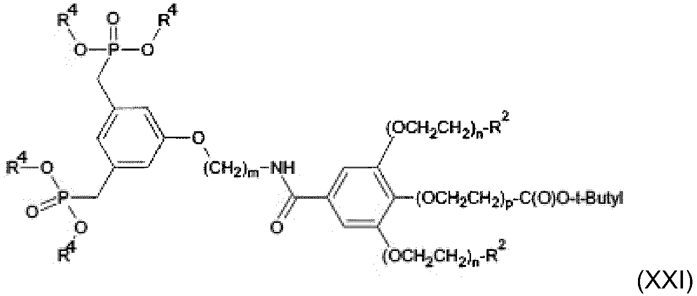
dans laquelle R⁴, R², m, n et p ont la même signification que dans la formule (I) ci-dessus et p a la même signification que dans la formule (I) et a une valeur qui est identique ou différente de la valeur de n dans le composé de formule (XIX) tel que défini ci-dessus et qui correspond à un composé particulier de formule (I) dans lequel R³ est un groupe -COOtBu ; et éventuellement ;
- la déprotection de la fonction carboxylique du composé de formule (XXI) ainsi obtenu pour donner la molécule dendritique correspondante de formule (I) dans laquelle R³ est un groupe -COOH.

13. Procédé selon la revendication 12, dans lequel des composés de formule (XX) sont préparés selon un procédé comprenant l'étape consistant à faire réagir du chlorure de tosyle avec un composé de formule (XXII) :
OHCH₂CH₂-(OCH₂CH₂)ₚ₋₁-C(O)O-*t*-butyle (XXII)
dans lequel p a la même signification que dans la formule (I) telle que définie dans la revendication 1.

14. Utilisation d'une molécule dendritique de formule (I) telle que définie dans l'une quelconque des revendications 1 à 8, en tant que support de médicament.

15. Molécule dendritique de formule (I) telle que définie dans l'une quelconque des revendications 1 à 8, en combinaison avec au moins un agent d'imagerie, pour son utilisation comme agent de contraste pour les plateformes d'imagerie médicale ou de diagnostic.
